# EUROPEAN PATENT SPECIFICATION

(11) **EP 3 429 569 B1**
(45) Date of publication and mention of the grant of the patent: **28.05.2025**
(21) Application number: 17767550.1
(22) Date of filing: 16.03.2017
(51) Int. Cl.: A61K 31/045, A61K 31/202, A61P 25/16, G01N 33/50

(54) **METHODS FOR PREVENTING OR TREATING PARKINSON'S DISEASE BY THE FARNESYLATION OF PARIS**
VERFAHREN ZUR VORBEUGUNG ODER BEHANDLUNG DER PARKINSON-KRANKHEIT DURCH DIE FARNESYLIERUNG VON PARIS
MÉTHODES DESTINÉES À PRÉVENIR OU À TRAITER LA MALADIE DE PARKINSON PAR FARNÉSYLATION DE PARIS

(30) Priority: 17.03.2016 US 201662309583 P
(43) Date of publication of application: 23.01.2019
(73) Proprietor: The Johns Hopkins University, Baltimore, MD 21201 (US); Research & Business Foundation Sungkyunkwan University, Suwon, Gyeonggi-do (KR)
(72) Inventor: SHIN, Joo-ho, Seoul 07980 (KR); JO, Areum, Suwon (KR); LEE, Yunjong, Chengju Choongchungbuk-do 361-540 (KR); DAWSON, Ted, M., Baltimore, MD 21212 (US); DAWSON, Valina, L., Baltimore, MD 21212 (US)
(74) Representative: Dehns
(86) International application number: PCT/US2017/022768
(87) International publication number: WO 2017/161155

(56) References cited:
- EP-A2- 3 000 464
- WO-A1-2015/042286
- WO-A1-91/16305
- WO-A2-2006/026785
- WO-A2-2012/067970
- JO AREUM ET AL: "PARIS farnesylation prevents neurodegeneration in models of Parkinson's disease", SCIENCE TRANSLATIONAL MEDICINE, vol. 13, no. 604, 28 July 2021 (2021-07-28), XP093090702, ISSN: 1946-6234, DOI: 10.1126/scitranslmed.aax8891
- TAKAHASHI ET AL.: "Dual action of isoprenols from herbal medicines on both PPAR.gamma. and PPAR.alpha. in 3T3-L1 adipocytes and HepG2 hepatocytes", FEBS LETT., vol. 514, no. 2-3, 13 March 2002 (2002-03-13), pages 315 - 322, XP004347719
- SHIN ET AL.: "PARIS (ZNF746) Repression of PGC-1.alpha. Contributes to Neurodegeneration in Parkinson's Disease", CELL, vol. 5, no. 144, 4 March 2011 (2011-03-04), pages 689 - 702, XP028185431

## Description

### STATEMENT OF GOVERNMENTAL INTEREST

This invention was made with government support under Grant No. NS38377, awarded by the National Institutes of Health. The government has certain rights in the invention.

### BACKGROUND OF THE INVENTION

Parkinson's disease (PD) is an incurable progressive neurodegenerative disease. It is characterized clinically by motor dysfunction that is due to the preferential loss of dopaminergic neurons in the substantia nigra (SN). Several genetic causes of PD have been identified, providing an opportunity to understand the molecular mechanisms underlying the disease process.

Current treatment strategies for PD are mainly limited to the management of the motor symptoms with drugs such as L-DOPA or dopamine receptor agonists and deep brain stimulation. Unfortunately, these therapies fail to prevent the progressive death of dopaminergic neurons (DA). In addition there are no proven therapies that delay or prevent the onset or progression of PD. WO 2015/042286 discloses the use of fenofibrate in the treatment of neurodegenerative diseases, such as Parkinson's disease.

Thus, there is a significant unmet need for new pharmacologic approaches to treat PD.

### SUMMARY OF THE INVENTION

Provided herein are methods of preventing or treating Parkinson's disease in subjects by administering drugs in effective amounts to cause the farnesylation of parkin interacting substrate (PARIS) and the enhanced expression of peroxisome proliferator-activated receptor-γ coactivator-1α (PGC-1α) in the brain. These methods alleviate the effects of Parkinson's disease in subjects, in part, by preventing the loss of dopamine neurons.

The present invention provides a composition comprising one or more inducers of PGC-1α for use in preventing or treating Parkinson's disease in a subject, wherein the inducer is an entity selected from the group consisting of Farnesol, ABT-0529 and a combination thereof, or a pharmaceutically acceptable salt, solvate, phosphate derivative, or stereoisomer thereof.

The structure of farnesol is 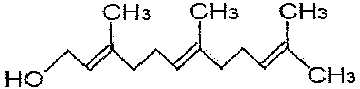 (2*E*,6*E*)-3,7,11-trimethyldodeca-2,6,10-trien-1-ol

The preferred inducer is farnesol, or a pharmaceutically acceptable salt, solvate, phosphate derivative, or stereoisomer thereof. The inducer may be administered orally, by injection, or topically. If farnesol is the inducer, it is preferred that farnesol is administered in an amount that results in an increase of the mRNA level of NRF-1 in the subject compared to when the subject is not administered farnesol. It is also preferred that farnesol is administered in an amount that increases farnesyl transferase activity in the brain of the subject compared to when the subject is not administered farnesol. It is also preferred that farnesol is administered in an amount that increases parkin interacting substrate (PARIS) farnesylation in the brain of the subject compared to when the subject is not administered farnesol. It is also preferred that the farnesylation occurs on a cysteine residue at residue 631 of PARIS. It is also preferred that farnesol substantially eliminates the PARIS binding to its target DNA in the subject when compared to a subject that is not administered farnesol. It is also preferred that the farnesol prevents the loss of dopamine neurons in the subject when compared to a subject that is not administered farnesol.

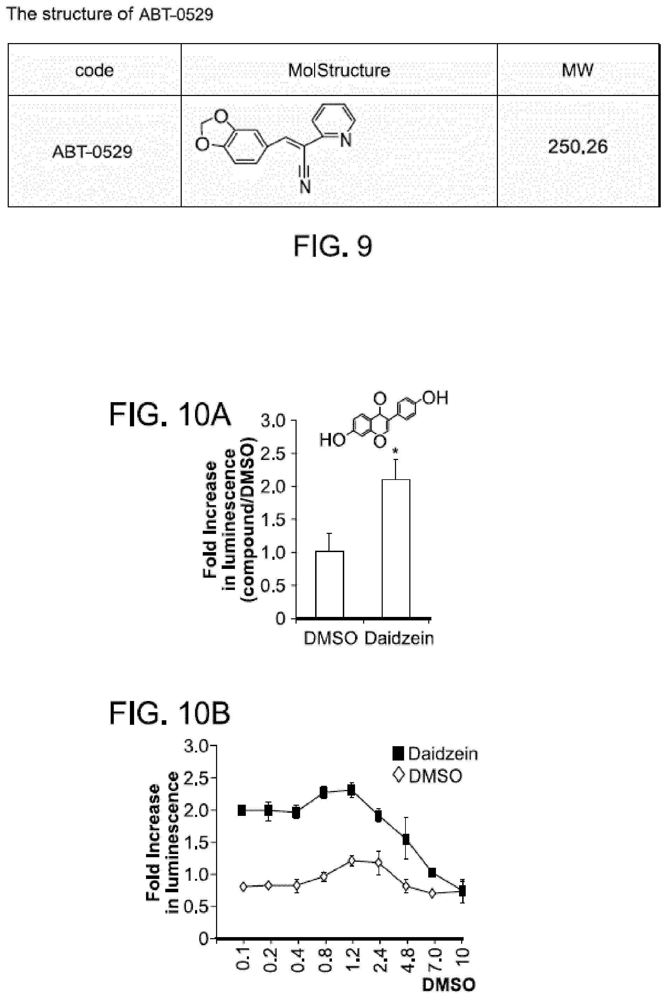

Please see FIG. 9 for the chemical structure of ABT-0529. It is preferred that the entity is administered in an amount that results in an increase of the mRNA level of NRF-1 in the subject compared to when the subject is not administered the entity. It is preferred that the entity is administered in an amount that increases farnesyl transferase activity in the brain of the subject compared to when the subject is not administered the entity. It is preferred that the entity is administered in an amount that increases PARIS farnesylation in the brain of the subject compared to when the subject is not administered the entity. It is preferred that the farnesylation occurs on a cysteine residue at residue 631 of PARIS. It is preferred that the entity substantially eliminates PARIS binding to its target DNA in the subject when compared to the subject that is not administered the entity. It is also preferred that the entity prevents the loss of dopamine neurons in the subject when compared to a subject that is not administered the entity.

### BRIEF DESCRIPTION OF THE DRAWINGS

Figure 1A -1D illustrates high throughput screening to identify compounds that elevate the expression of PGC-1α.
Figure 2A-2J illustrates compounds that activate PGC-1α promoter in the presence of PARIS.
Figure 3A-3J illustrates the 9 compounds that activate the PGC-1α promoter in the presence of PARIS were evaluated to determine which compounds induce PGC-1α levels in SH-SY5Y cells
Figure 4A-4H illustrates the farnesylation site of PARIS.
Figure 5A-5I illustrates the protection of dopaminergic neurons by farnesol.
Figure 6A-6K illustrates the protective effects of farnesol.
Figure 7A-7G illustrates that farnesylation of PARIS is required for the protective effects of farnesol in-vivo.
Figure 8 illustrates the method of action of farnesol as determined by the present invention.
Figure 9 illustrates the chemical structure of ABT-0529.
Figure 10A-10D illustrates an assay used in the present invention was robust for high-throughput screening (HTS).
Figure 11 illustrates the high-throughput screening protocol used in the present invention.
Figure 12 illustrates sixteen of the seventeen compounds identified from the primary screen.
Figure 13A-F illustrate real-time qRT-PCR shows that FXR mRNA is strongly observed in liver, kidney, colon, and ovary along with mild expression in spleen, trachea, esophagus, testis, and prostate.
Figure 14A and 14B illustrates farnesol abolished the level of chromatin-bound PARIS WT but not PARIS C631S, while there is no change in the distribution of PARIS in the cytoplasmic, membrane, and soluble nuclear fractions.
Figure 15A to 15D illustrates co-immunostaining analysis indicates that farnesol led to increased dopaminergic neuronal PGC-1α immunoreactivity

### DETAILED DESCRIPTION OF THE INVENTION

Embodiments of the disclosure concern compositions comprising one or more inducers of PGC-1α for use in treating or preventing Parkinson's disease, wherein the inducer is an entity selected from the group consisting of Farnesol, ABT-0529 and a combination thereof, or a pharmaceutically acceptable salt, solvate, phosphate derivative, or stereoisomer thereof.

In certain embodiments, the level to which an entity induces PGC-1α expression may be any level so long as it provides amelioration of at least one symptom of the neurological disorder, including PD. The level of expression may increase by at least 2, 3, 4, 5, 10, 25, 50, 100, 1000, or more fold expression compared to the level of expression in a standard, in at least some cases. An individual may monitor expression levels of PGC-1α using standard methods in the art, such as Northern blot analysis or quantitative PCR, for example.

An individual known to have PD, suspected of having PD, or at risk for having PD may be provided an effective amount of a composition of the invention. Those at risk for PD may be those individuals having one or more genetic factors, may be of advancing age, and/or may have a family history, for example.

In particular embodiments of the disclosure, an individual is given an agent for PD therapy in addition to the composition of the invention. Such additional therapy may include L-DOPA or dopamine receptor agonists and/or deep brain stimulation, for example. When combination therapy is employed with the composition , the additional therapy may be given prior to, at the same time as, and/or subsequent to the composition.

Prior to the use of the compositions of the disclosure, the diagnosis of PD may occur by any methods or means, including at least genetic marker assay, single-photon emission computed tomography, olfactory system testing, autonomic system testing, or a combination thereof.

Parkin substrate, PARIS (ZNF746), whose levels accumulate in models of parkin inactivation and in human PD brain were previously identified. Through occupation of insulin response sequences (IRSs) in the proliferator-activated receptor-γ coactivator-1α (PGC-1α) promoter, PARIS transcriptionally represses the expression of PGC-1α and its target gene, nuclear respiratory factor 1 (NRF-1). PARIS is required for progressive loss of dopaminergic neurons in conditional knockout of parkin in adult animals and overexpression of PARIS leads to the selective DA neuronal degeneration. Accompanying the DA neuronal degeneration are PARIS-dependent declines in mitochondrial mass and respiration suggesting that parkin loss impairs mitochondrial biogenesis, consistent with a defect in PGC-1 α.

PGC-1α is a master regulator of mitochondrial function through co-regulating transcriptional programs important for mitochondrial biogenesis and protecting against mitochondrial oxidative stress. Emerging evidence suggests that PGC-1α plays a role in PD. PGC-1α levels are decreased in PD patients. PGC-1α downregulation in PD may be due to PGC-1α promoter methylation. PGC-1α knockout mice are more susceptible to the degenerative effects of the PD neurotoxin 1-methyl-4-phenyl-1,2,3,6-tetrahydropyridine (MPTP) and overexpression of PGC-1α protects against N-methyl-4-phenylpyridinium ion (MPP⁺) toxicity, the active metabolite of MPTP. In addition, overexpression of PGC-1α protects against α-synuclein, MPTP, oxidative stress and rotenone-induced degeneration. The age of onset and risk of PD is associated with polymorphisms in PGC-1α and in parkin associated PD, PGC-1α is dysfunctional. PGC-1α responsive genes are down regulated in dopaminergic neurons from PD patients suggesting that it plays an important role in PD pathogenesis. The loss of dopamine neurons when PARIS is overexpressed is prevented by PGC-1α overexpression, suggesting PGC-1α is the primary target linking PARIS to dopaminergic neuronal degeneration. Thus, defects in PGC-1α signaling are emerging as important contributors to dopaminergic degeneration in PD and the regulation of PARIS by parkin may be the underlying mechanism linking PGC-1α to PD.

Since the parkin-PARIS-PGC-1α pathway seems to play an important role in the death of dopaminergic neurons in PD, an unbiased screen was developed and conducted to identify compounds that maintain PGC-1α function in the presence of elevated PARIS. The present invention has shown that CSU-1806 (farnesol) is a potent inducer of PGC-1α via farnesylation of PARIS, which inhibits its transcriptional repression of PGC-1α. Moreover, farnesol was demonstrated to profoundly prevent the loss of dopamine neurons in PARIS transgenic mice and adult conditional parkin knockout mice.

### High-throughput screening for inducers of PGC-1α

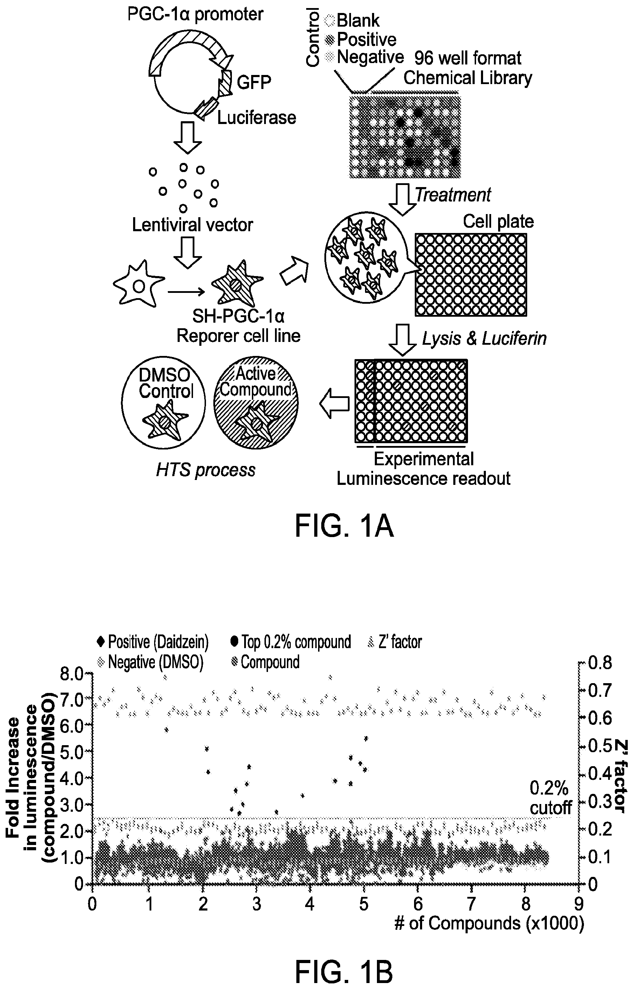

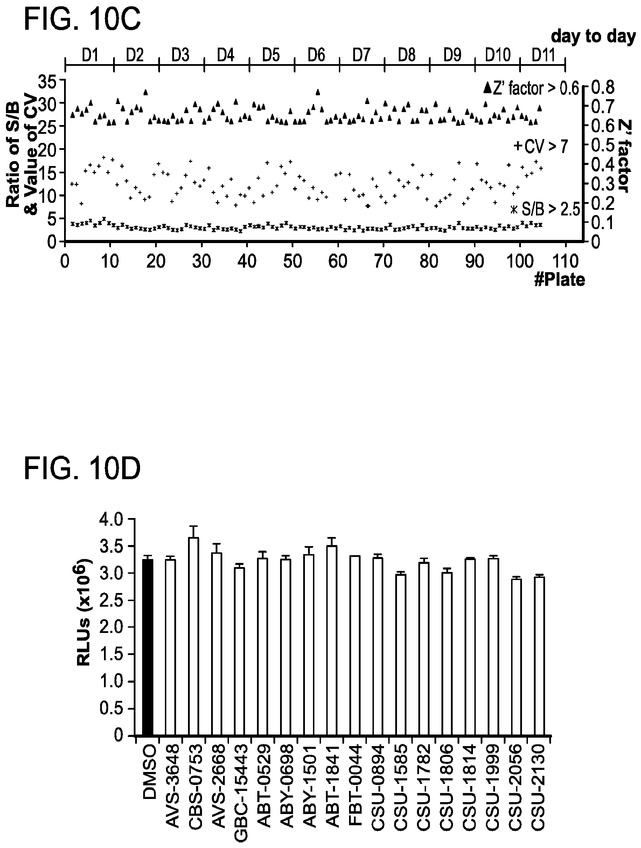

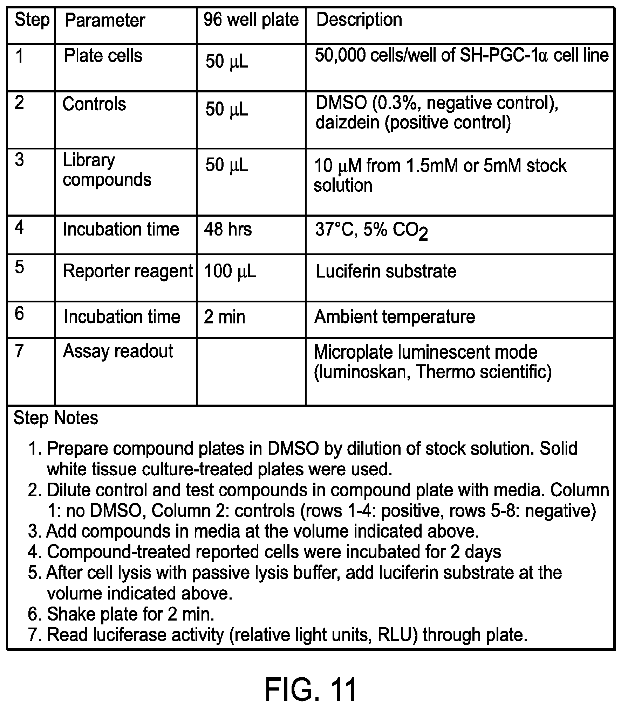

To identify compounds that elevate the expression of PGC-1α, a stable reporter cell line that expresses luciferase and destabilized GFP (dscGFP) under control of the 1 Kb human PGC-1α promoter (SH-PGC-1α) was generated (Figure 1A). A representative chemical library was selected from 230,000 compounds based on their structural, pharmacokinetic, and pharmacodynamics properties (Korean Chemical Bank, Daegeon). This representative chemical library contained 6000 pharmacologically active compounds that were quality-controlled by liquid chromatography mass spectroscopy (LC-MS). Additionally the Spectrum Collection (Microsource, (www)msdiscovery.com/spectrum.html) containing 2320 compounds comprised of clinically approved drugs (60%), natural products (25%), and other bioactive components (15%) was also used for screening. To determine whether the assay was robust for high-throughput screening (HTS), standard parameters were determined, including plate-to-plate and day-to-day variations, signal-to-background (S/B) ratio, coefficient of variation (CV) and Z' factor. Variations within and between plates and between days were very minimal (Figure 10A-10C). The Z' factors were within the 0.5 - 1 range, commonly taken as index of assay readiness for HTS. DMSO up to 0.5% had no effect on assay sensitivity and cell viability (Figure 10B and 10D). All HTS procedures were performed according to NIH guidelines (High-Throughput Screening Assay Guidance Criteria,
(www)ncats.nih.gov/research/reengineering/ncgc/assay/criteria/criteria.html) (Figure 11: HTS assay protocol).

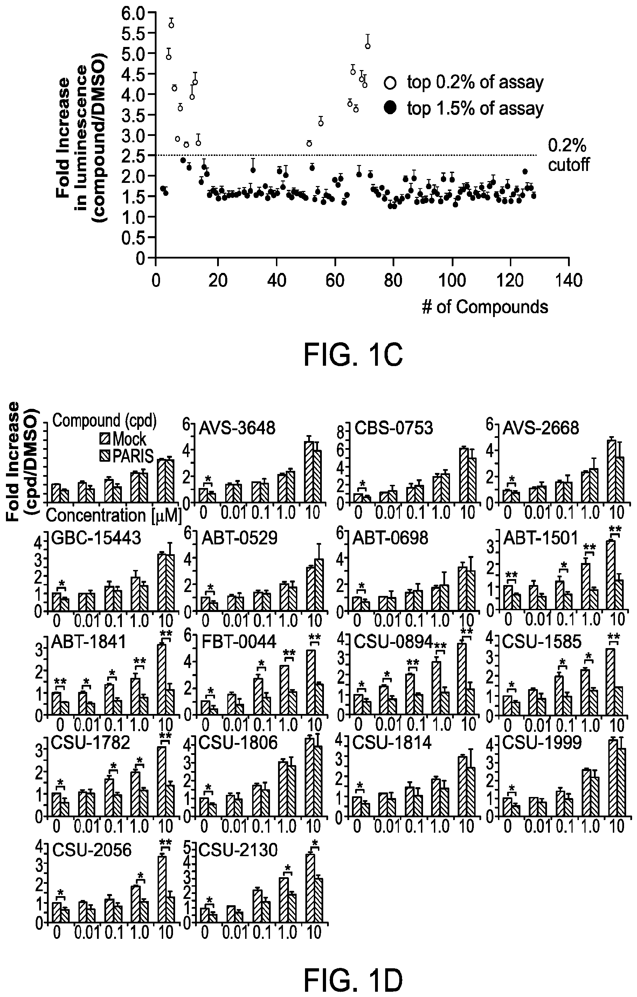

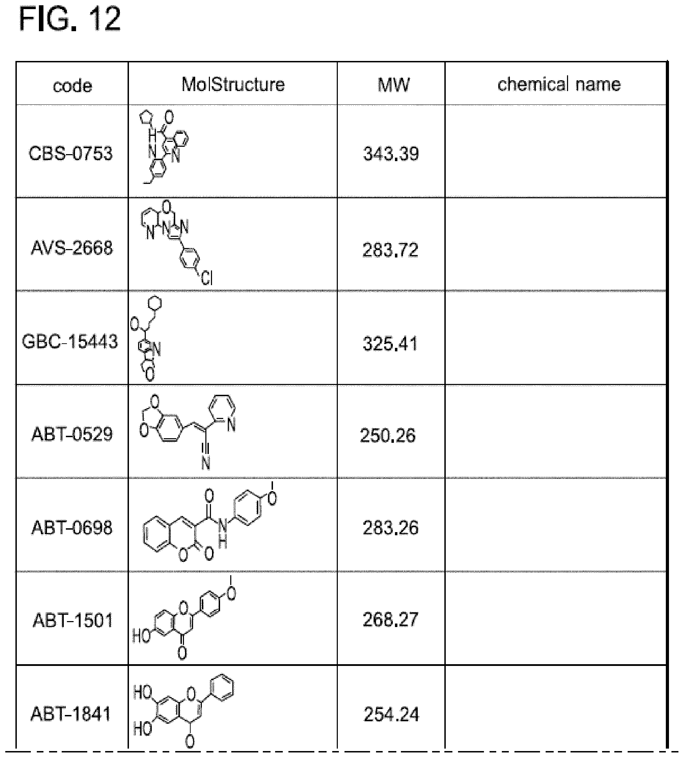

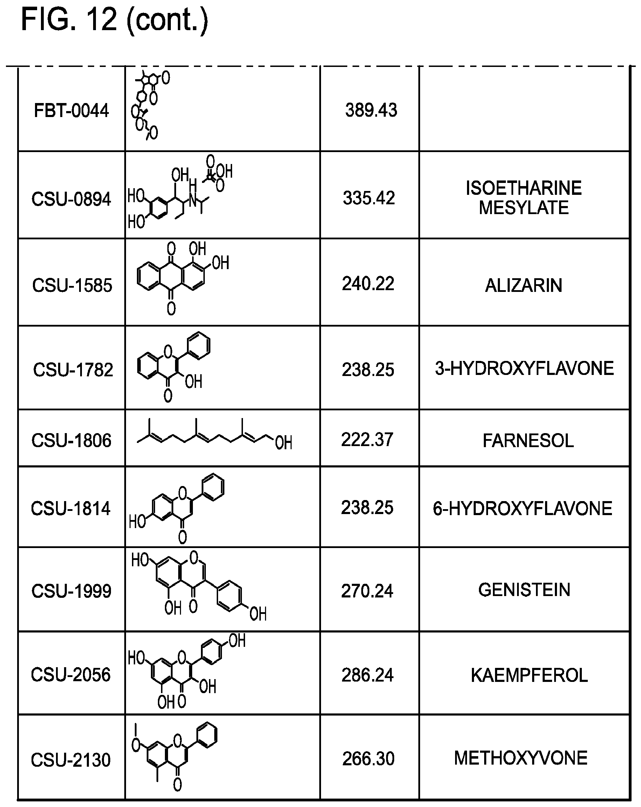

SH-PGC-1α cells were treated with each of the 8320 compounds at a final concentration of 10 µM for 48 hours. Luciferase activity was assessed as the primary read-out and normalized to DMSO (Figure 1A). In the initial screen, 128 compounds increase luciferase activity 1.5-fold (Figure 1B). These compounds were rescreened in triplicate (Figure 1C). All compounds increase luciferase activity and of these compounds 17 compounds increase luciferase activity 2.5-fold or greater (Figure 1C). None of these 17 compounds show cellular toxicity at the working concentration of 10 µM (Figure 10D and Figure 12). These 17 compounds were evaluated to identify activators of PGC-1α in the setting of PARIS overexpression (Figure 1D). PARIS or Mock transfected SH-PGC-1α cells were exposed to increasing concentrations (0, 0.01 µM, 0.1 µM, 1 µM, and 10 µM) of the individual compounds. 48 hours later luciferase activity was monitored. Equal expression of PARIS and suppression of PGC-1α was confirmed in each setting. Nine of the 17 compounds prevented the suppression of PGC-1α in presence of PARIS and activated the PGC-1α promoter in a dose-dependent manner (Figure 1D). To assess whether any of these 9 compounds have potential for central nervous system (CNS) application, ADME properties (absorption, distribution, metabolism and excretion) were assessed by StarDrop^{™} ((www)optibrium.com/stardrop/), revealing that AVS-3648, ABT-0529, and CSU-1806 (farnesol) possess a high likelihood of blood-brain barrier (BBB) permeability and no interaction with p-glycoprotein transport (P-gp).

### Farnesol (CSU-1806) inhibits PARIS to induce PGC-1α

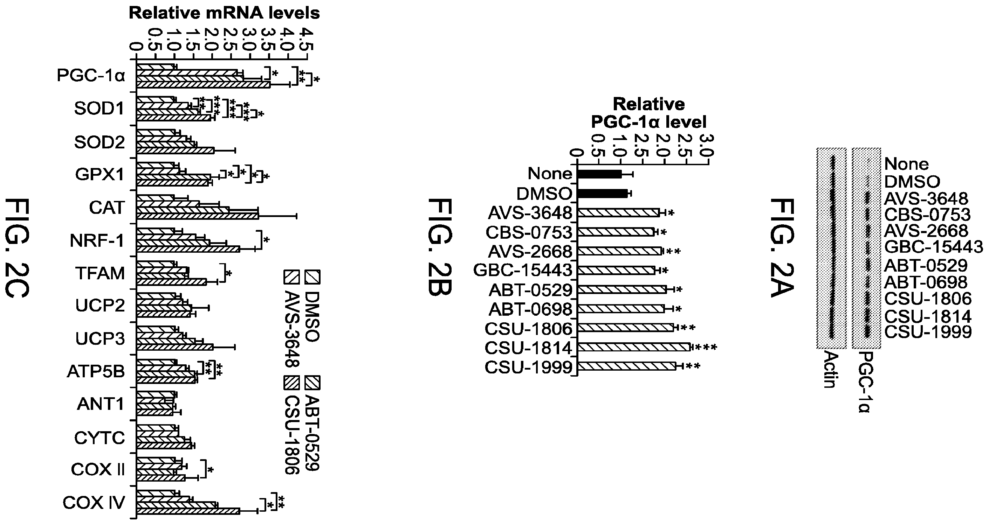

The 9 compounds that activate the PGC-1α promoter in the presence of PARIS were evaluated to determine which compounds induce PGC-1α levels in SH-SY5Y cells (Figure 2A and 2B). PGC-1α is significantly increased by the potential CNS penetrant drugs, AVS-3648, ABT-0529, and farnesol (CSU-1806) as determined by immunoblot analysis (Figure 2A and 2B). AVS-3648, ABT-0529, and farnesol (CSU-1806) were utilized to determine whether the increase in PGC-1α functionally regulates PGC-1α target genes involved in oxidant metabolism, mitochondrial biogenesis, and oxidative phosphorylation. The mRNA level of PGC-1α target genes including copper/zinc superoxide dismutase (SOD1), manganese SOD (SOD2), glutathione peroxidase (GPx1), catalase (CAT), nuclear respiratory factor-1 (NRF-1), mitochondrial transcription factor A (TFAM), mitochondrial uncoupling proteins (UCP2 and UCP3), and the oxidative phosphorylation regulators, ATP5b, cytochrome C (CytC) and cytochrome C oxidase (COX II and IV) were determined by real-time quantitative RT-PCR (qRT-PCR) (Figure 2C). PGC-1α, SOD1, and ATP5B mRNA levels are increased following treatment with AVS-3648, ABT-0529, or farnesol (CSU-1806) (Figure 2C). Interestingly, only farnesol (CSU-1806) robustly increased the mRNA level of NRF-1, which was identified as a potential PARIS/PGC-1α target gene in PD models.

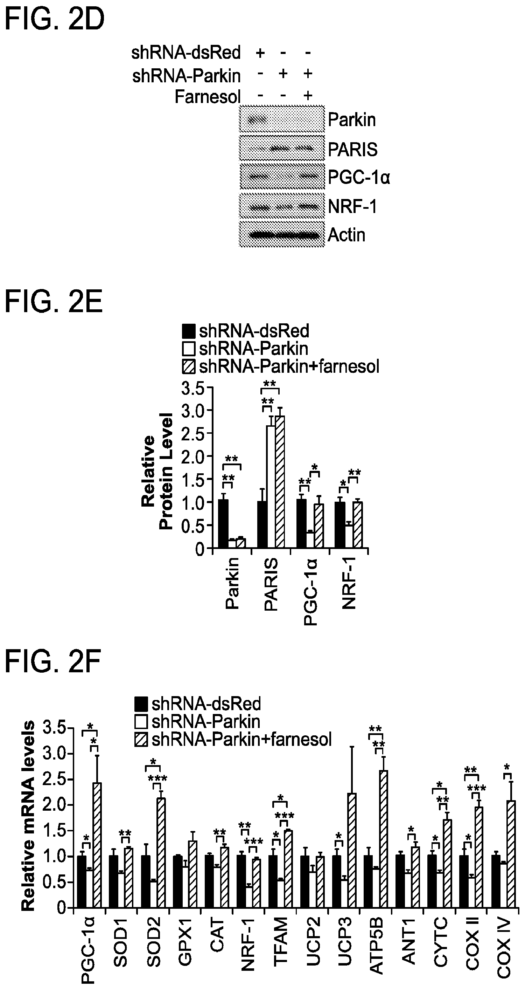

To determine whether farnesol induces PGC-1α in the setting of loss of parkin, lentiviral shRNA-parkin was used to reduce parkin expression in SH-SY5Y cells as previously described. Knockdown of parkin leads to a 2.7 fold increase in the level of PARIS and a 65% decrease of PGC-1α (Figure 2D and 2E). Farnesol restores the level of PGC-1α without affecting the level of parkin and PARIS (Figure 2D and 2E). The mRNA levels of PGC-1α and its target genes were assessed after knocking down parkin by real-time qRT-PCR. As previously described, the mRNA of PGC-1α and NRF-1 are decreased in parkin knockdown SH-SY5Y cells and this reduction is rescued by farnesol (Figure 2F). Furthermore, the mRNA levels of SOD2, TFAM, ATP5B, CYTC, and COX II in the setting of parkin knockdown are significantly increased in the presence of farnesol (Figure 2F). Taken together, these results indicate that farnesol restores the level of PGC-1α and NRF-1 in the absence of parkin.

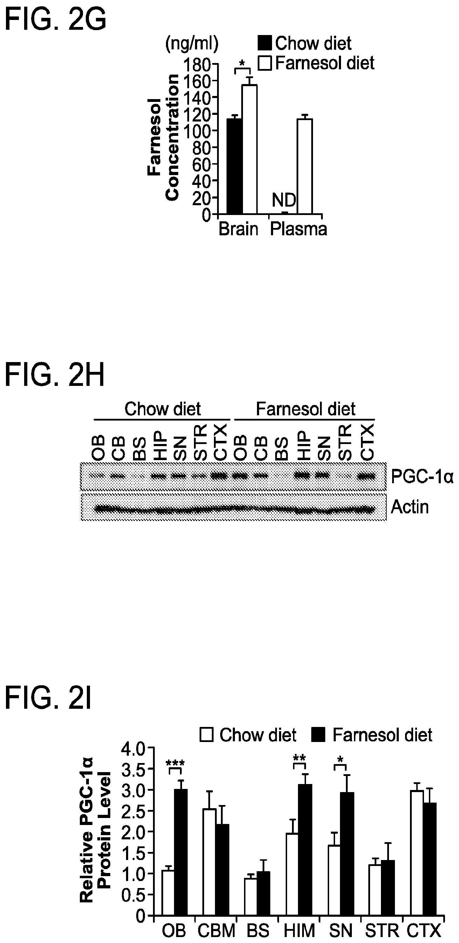

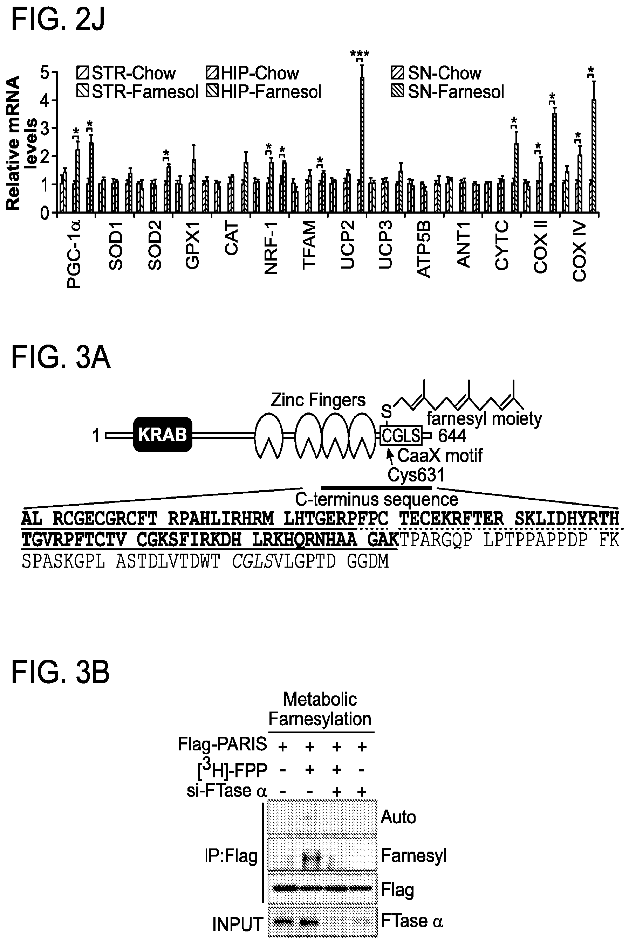

Farnesol is a natural organic compound in both plants and animals. Phosphate derivatives of farnesol are the building blocks of cholesterol in mammalian cells. In order to determine whether exogenous farnesol penetrates the CNS, mice were fed a farnesol diet (0.5% w/w of trans-farnesol in AIN-76A diet, Research diet, NJ) for 7 days and the concentration of farnesol was measured in the brain and plasma by mass spectrometry (Figure 2G). Plasma levels of farnesol increase to 113 ng/ml in plasma from non-detectable levels in farnesol fed mice versus control diet (AIN-76A) fed mice. Farnesol levels in the brain significantly increase by 37% to 155 ng/g of farnesol in farnesol fed mice versus control diet fed mice (Figure 2G). The level of PGC-1α was measured from different brain regions in farnesol-fed mice. PGC-1α levels increase 2.7-fold in the olfactory bulb (OB), 1.6-fold in hippocampus (HIP), and 1.7-fold in substantia nigra (SN) as compared to that of mice on normal mouse chow (Figure 2H and 2I). No significant change in PGC-1α levels are observed in the cerebellum (CBM), brain stem (BS), striatum (STR), or frontal cortex (CTX) (Figure 2H and 2I). These data suggest that the alteration of PGC-1α by farnesol might be brain region specific. In addition, mRNA levels of PGC-1α and a variety of PGC-1α's target genes in the STR, HIP, and SN of farnesol-fed mice were measured. Since farnesol did not affect PGC-1α levels in the STR, it was used as a negative control. The mRNA level of PGC-1α is significantly upregulated in the HIP and SN, but not in the STR of farnesol fed mice as compared to normal chow fed mice, indicating that the increased protein level of PGC-1α in the HIP and SN is mainly due to a transcriptional process (Figure 2J). Interestingly, the mRNA levels of NRF-1, COX II, and COX IV are upregulated in both HIP and SN of farnesol fed mice compared to normal chow fed mice (Figure 2J). However, mRNA levels of SOD2, TFAM, UCP2, and CYTC are exclusively increased in SN, but not in the HIP and STR of farnesol fed mice. These findings suggest that farnesol regulates the level of PGC-1α along with a different subset of PGC-1α's target genes in a brain region specific manner.

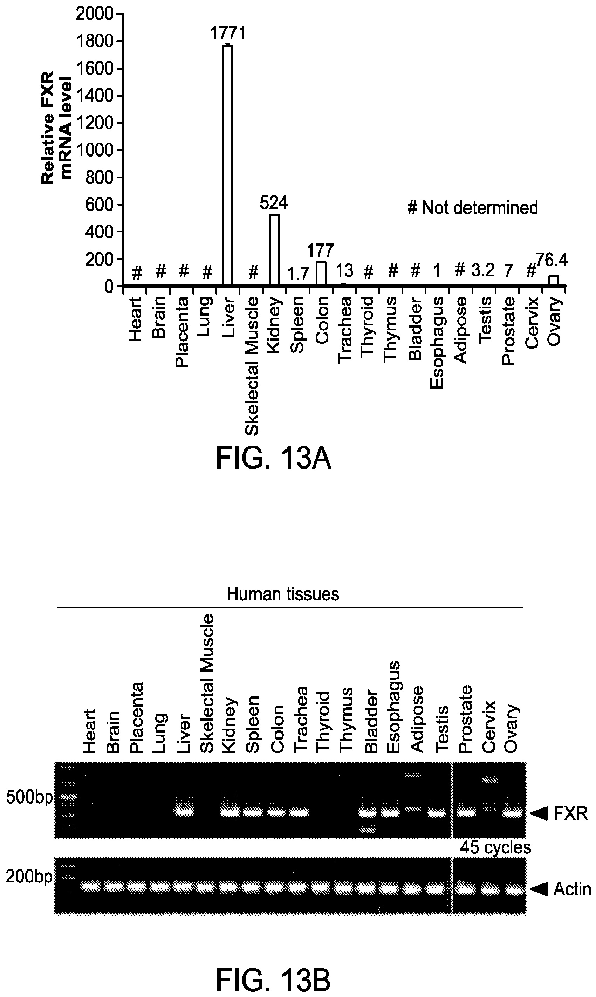

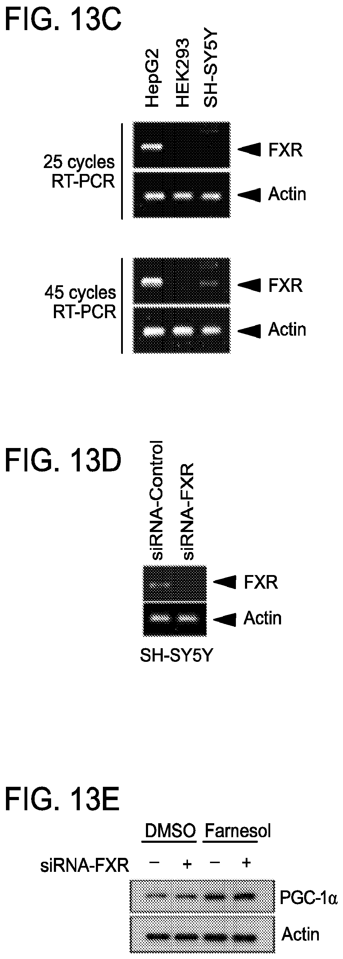

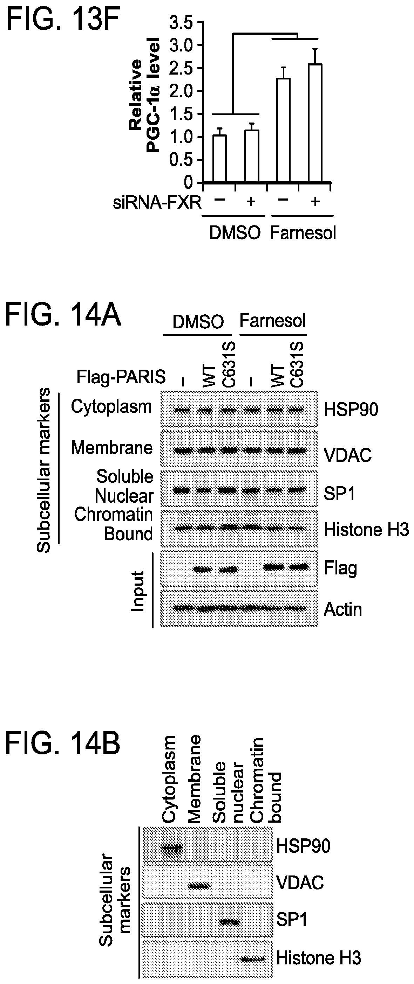

Farnesol is known to activate the farnesoid X receptor (FXR), which is found primarily in liver, kidney, and intestine. To determine if FXR is expressed in brain, the mRNA of FXR was determined by real time qRT-PCR in diverse human tissues including heart, brain, placenta, lung, liver, skeletal muscle, kidney, spleen, colon, trachea, thyroid, thymus, bladder, esophagus, adipose, testis, prostate, cervix, and ovary (Master panel II^{™}, Clontech). As previously reported, real-time qRT-PCR shows that FXR mRNA is strongly observed in liver, kidney, colon, and ovary along with mild expression in spleen, trachea, esophagus, testis, and prostate (Figure 13). As previously reported, FXR mRNA is not detected in brain, heart, placenta, lung, skeletal muscle, thyroid, thymus, bladder, adipose, and cervix. To determine if there are small levels of FXR mRNA in these tissues, the RT-PCR was subjected to 45 cycles at saturation, FXR was only observed in one more tissue, the bladder. FXR mRNA expression was evaluated in HepG2 cells (liver-origin), HEK293 cells (kidneyorigin), and SH-SY5Y cells (neuronal origin). Robust mRNA expression of FXR is found in HepG2 but not in HEK293 and SH-SY5Y cells. However, the mRNA expression of FXR was detected in SH-SY5Y at 45 cycles of semi-quantitative RT-PCR. To test if there is a possible relationship between FXR and PGC-1α's induction in farnesol-treated SH-SY5Y cells, siRNA to FXR was used to knockdown FXR. In the setting of FXR knockdown, farnesol treatment has no effect on PGC-1α levels. These findings suggest that farnesol induction of PGC-1α is FXR independent.

To confirm that the increase in PGC-1α and NRF-1 levels by farnesol is PARIS dependent, farnesol was administered to SH-SY5Y cells following shRNA knockdown of PARIS. As previously described knockdown of PARIS leads to greater than 2 fold increase in PGC-1α and NRF-1 levels. Farnesol fails to enhance the levels further suggesting that PARIS is required for farnesol elevation of PGC-1α and NRF-1 levels. Since, farnesol is also capable of activating both PPARα and PPARγ *in vitro,* the levels of PPARα and PPARγ and some of their target genes were assessed. Farnesol fails to influence the levels of PPARα and PPARγ protein and mRNA or some of their target genes acyl-coenzyme A oxidase 1 (ACOX1), carnitine O-palmitoyltransferase 1 (CPT-1A), medium-chain specific acyl-CoA dehydrogenase (MCAD) and long-chain specific acyl-CoA dehydrogenase (LCAD). Interestingly, the mRNA PPARγ levels decrease and ACOX1 and CPT-1A are upregulated in the absence of PARIS, suggesting that these genes might be PARIS targets. There is also no alteration in the levels of PPARα and PPARγ in farnesol fed mice, while PGC-1α and NRF-1 levels increase. Moreover, farnesol treatment decreases the mRNA levels of PPARγ and ACOX1 and increases the levels of MCAD in the SN of farnesol fed mice. Farnesol also fails to activate a PPAR responsive element in SH-SY5Y cells. Taken together these findings suggest that the effects of farnesol in the brain are independent from PPARα and PPARγ and require PARIS.

### Farnesylation of PARIS transcriptionally regulates PGC-1α

Farnesyl transferase (FTase) utilizes farnesol and its intermediates for protein farnesylation via modification on cysteine residues at carboxyl-termini with a CaaX motif. Farnesylation is a form of post-translational lipidation of proteins with isoprenoids that is designated protein prenylation. Bioinformatic analysis reveals a putative CaaX motif at the C-terminus of PARIS at CGLS (amino acids 631 to 634) (Figure 3A). FTase recognizes cysteine in the context of the CaaX consensus, where C is cysteine, A is an aliphatic amino acid, and X is a C-terminal amino acid of target protein. FTase prefers methionine or serine at the X position and transfers a 15-carbon farnesyl group. The CGLS sequence is highly conserved among the predicted isoforms of PARIS and between species (human, mouse, rat, cow, and gorilla). In order to determine whether PARIS is farnesylated, [³H]-farnesyl pyrophosphate (FPP) treated SH-SY5Y cells were transfected with Flag-tagged PARIS plus either siRNA-control or siRNA to FTase α. Autoradiography shows that immunoprecipitated Flag-tagged PARIS is farnesylated, whereas SH-SY5Y cells with knockdown of FTase α eliminated the farnesylation of PARIS (Figure 3B). Immunoblot analysis with an α-farnesyl antibody confirms the farnesylation of PARIS in [³H]- FPP treated SH-SY5Y cells and knockdown of FTase α reduces the farnesylation of PARIS (Figure 3B).

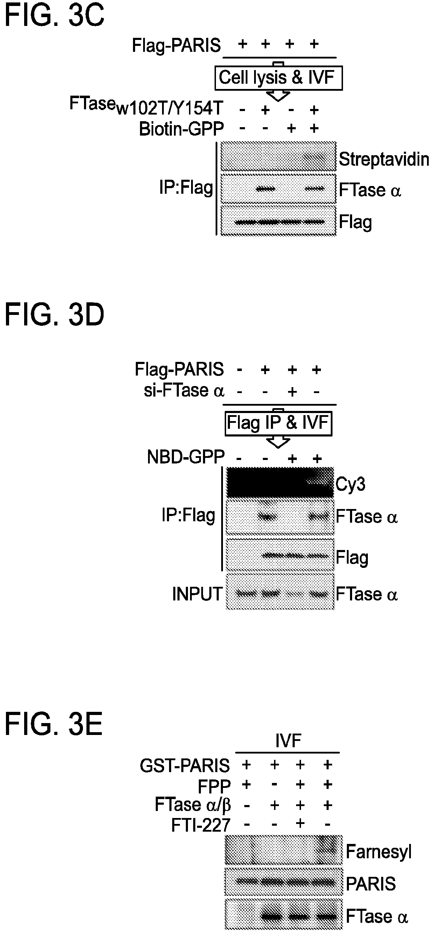

Next, a biotechnological approach to confirm that PARIS is prenylated was applied (Nguyen et al., 2009). The total cell lysate of SH-SY5Y cells transfected with Flag-tagged PARIS was incubated with a biotin-functionalized geranyl pyrophosphate analogue (Biotin-GPP) and the recombinant FTase W102T/Y154T mutant, which was engineered to utilize Biotin-GPP as a lipid donor (Nguyen et al., 2009). After the immunoprecipitation of Flag-tagged PARIS, Biotin-GPP-conjugated PARIS is detected by an α-streptavidin antibody, indicating that PARIS is a substrate for FTase prenylation (Figure 3C). In addition, the immunoprecipitate of Flag-tagged PARIS from SH-SY5Y cells transfected with either siRNA-control or siRNA-FTase α was mixed with NBD-GPP, a Cy3 fluorescent analog of FPP for an *in vitro* farnesylation assay (IVF). NBD-GPP fluorescent signal is observed in the immunoprecipitated Flag-tagged PARIS, demonstrating that endogenous FTase co-immunoprecipitates with Flag-tagged PARIS and the immunoprecipitated FTase successfully transferred NBD-GPP to PARIS (Figure 3D). To determine whether FTase directly farnesylates PARIS, recombinant GST-PARIS was incubated with FTase and FPP in the presence and absence of the FTase inhibitor, FTI-277. GST-PARIS undergoes farnesylation by FTase, which is abolished by the addition of FTase inhibitor, FTI-277 (Figure 3E).

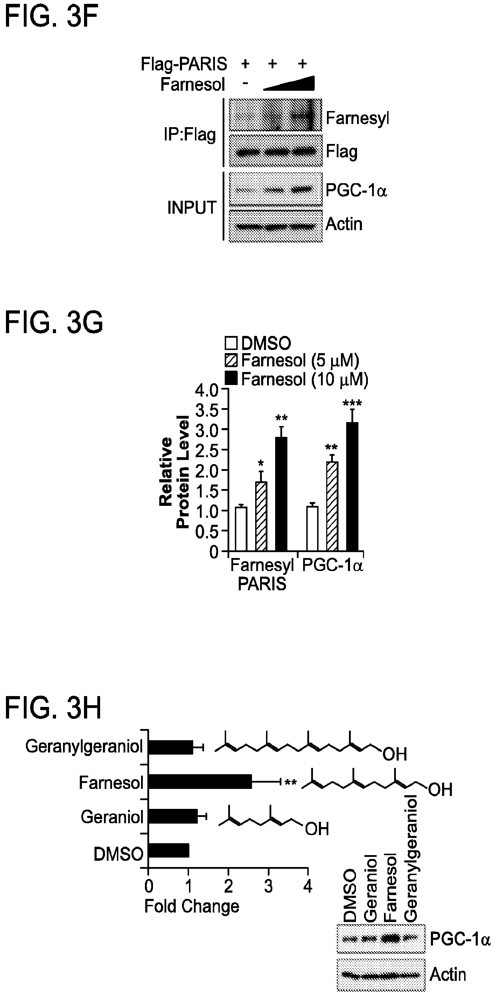

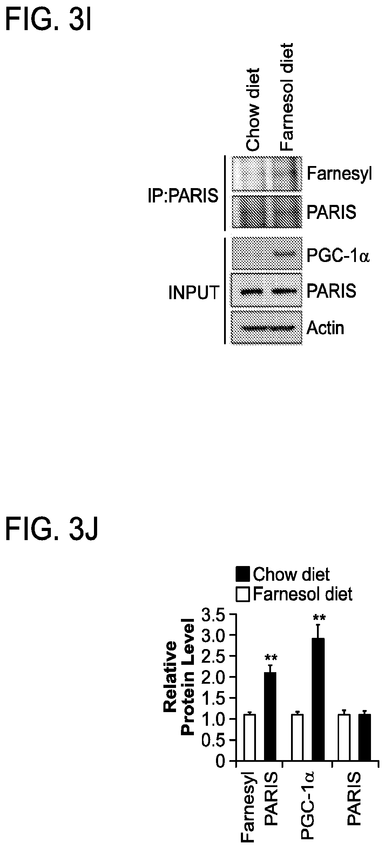

To ascertain whether farnesol leads to enhanced farnesylation of PARIS, SH-SY5Y cells were transfected with Flag-tagged PARIS and subjected to farnesol treatment for 48 hours followed by immunoprecipitation (Figure 3F). Farnesol increases PARIS farnesylation in a dose-dependent manner (Figure 3F and 3G). Accompanying the increase in PARIS farnesylation is an increase in PGC-1α (Figure 3F and 3G). To ascertain the specificity of farnesol, geraniol or geranylgeraniol were utilized since they have similar structures to farnesol. Geraniol or geranylgeraniol have no effect on PGC-1α levels (Figure 3H). To determine whether farnesol increases PARIS farnesylation *in vivo,* PARIS was immunoprecipitated from the SN of farnesol fed mice. Immunoblot analysis shows that endogenous PARIS is farnesylated and farnesol enhances the level of farnesylated PARIS along with the upregulation of PGC-1α (Figure 3I and 3J).

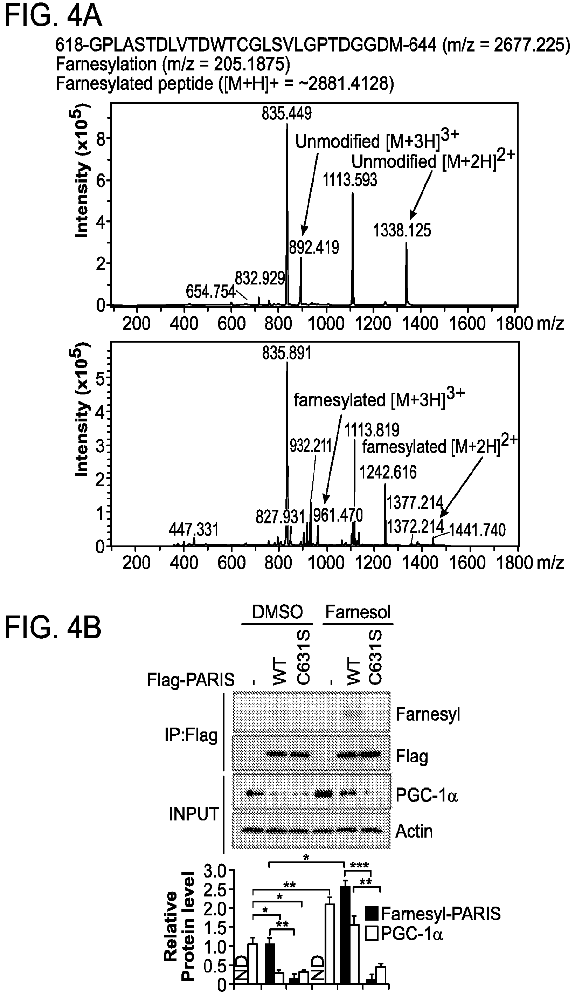

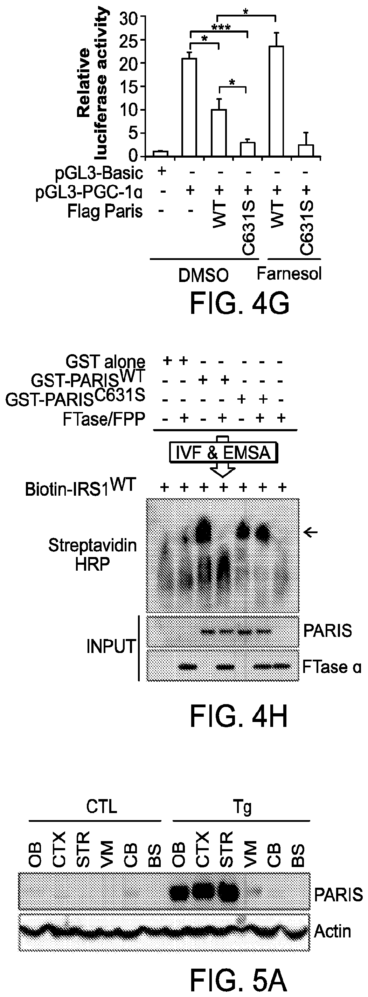

To confirm that the CGLS (amino acids 631 to 634) of PARIS contain the farnesylation site, farnesylated PARIS by IVF was subjected to mass spectrometry (microTOF-Q and HCT ultra ion-trap) (Figure 4A). Since the peptide (⁶¹⁸GPLASTDLVTDWT**C**GLSVLGPTDGGDM⁶⁴⁴) contains highly acidic amino acids and a hydrophobic lipid moiety, ionic fragmentation for MS/MS was disturbed and as such we were only able to confirm that the farnesyl group exists on the ⁶¹⁸GPLASTDLVTDWTCGLSVLGPTDGGDM⁶⁴⁴ peptide via increased molecular weight consistent with the addition of a farnesyl moiety. Since this peptide harbors only one cysteine residue at residue 631 it is highly probably that this is the site of farnesylation of PARIS (Figure 4A). To confirm that residue 631 is the site of farnesylation, a conserved C631S mutation of PARIS was generated.

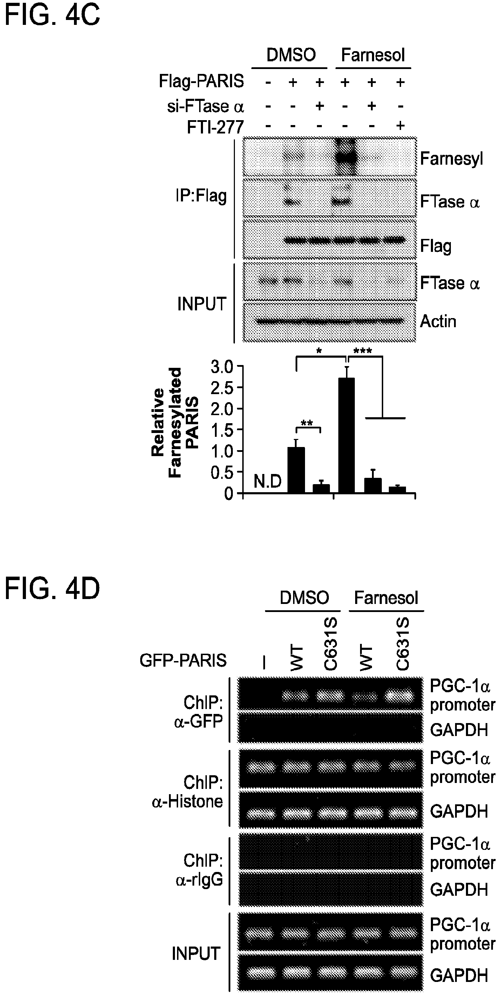

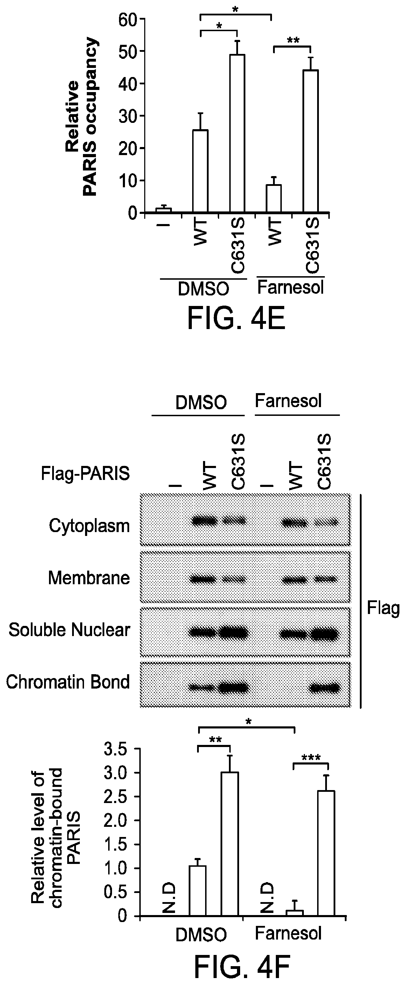

FLAG-PARIS WT or FLAG-PARIS C631S mutant were immunoprecipitated and the level of farnesylation was determined in the presence of farnesol. PARIS WT is farnesylated and its farnesylation is increased with farnesol treatment, whereas there is no farnesylation of the PARIS C631S mutant (Figure 4B). Accompanying the farnesylation of PARIS WT by farnesol is an upregulation of PGC-1α (Figure 4B). Whereas farnesol has no effect on the restoration of PGC-1α levels in the setting of the PARIS C631S mutant (Figure 4B). To confirm that farnesylation of PARIS requires FTase, siRNA-FTase α knockdown or FTI-277 inhibition of FTase in the setting of PARIS overexpression was performed (Figure 4C). FTase α knockdown or FTI-277 inhibition strongly prevents farnesylation of PARIS by farnesol (Figure 4C). To ascertain whether the farnesylation of PARIS affects the DNA binding activity of PARIS on the IRS motif of the *PGC-1α* promoter, a chromatin immunoprecipitation assay (ChIP) was performed from SH-SY5Y transfected with GFP-tagged PARIS WT or GFP-tagged PARIS C631S. Farnesol reduces the chromatin occupancy of PARIS WT but not PARIS C631S on the *PGC-1α* promoter (Figure 4D and 4E). To examine the possibility that the farnesylation of PARIS alters its subcellular distribution, subcellular fractionation was used to monitor the levels of PARIS in cytosolic, membrane, soluble nuclear, and chromatin-bound nuclear fractions by immunoblot analysis (Figure 4F). Consistent with the ChIP assay, farnesol abolished the level of chromatin-bound PARIS WT but not PARIS C631S, while there is no change in the distribution of PARIS in the cytoplasmic, membrane, and soluble nuclear fractions (Figure 4F and Figure 14). Accompanying the farnesylation of PARIS WT by farnesol treatment is an increase of *PGC-1α* promoter activity that is not observed with the PARIS C631S mutant (Figure 4G). An electrophoretic mobility shift assay (EMSA) shows that *in vitro* farnesylated GST-tagged PARIS WT fails to bind to the insulin responsive sequence (IRS) in the *PGC-1α* promoter, whereas naive GST-tagged PARIS WT and C631S form DNA-protein complexes. Taken together these data indicated that PARIS is farnesylated and farnesylation of PARIS eliminates its DNA binding affinity thereby preventing its suppression of PGC-1α.

### Farnesol protects against loss of dopaminergic neurons in models of PD

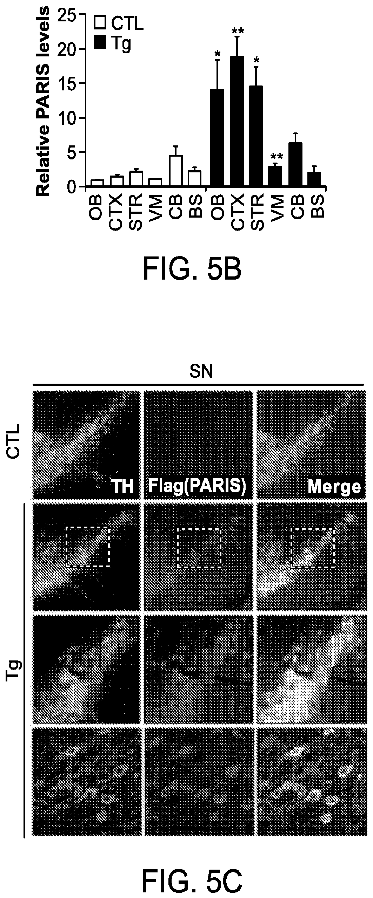

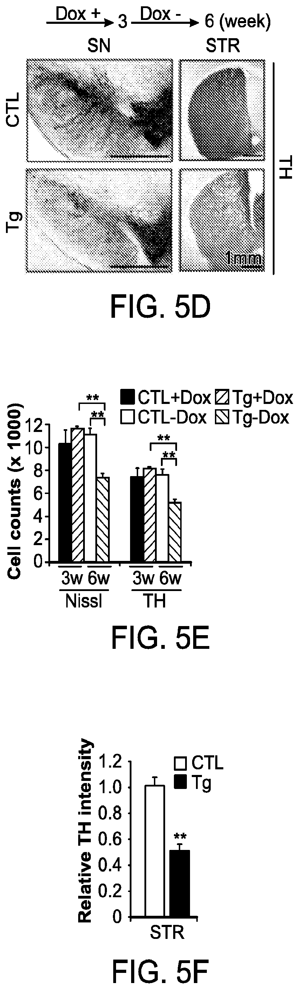

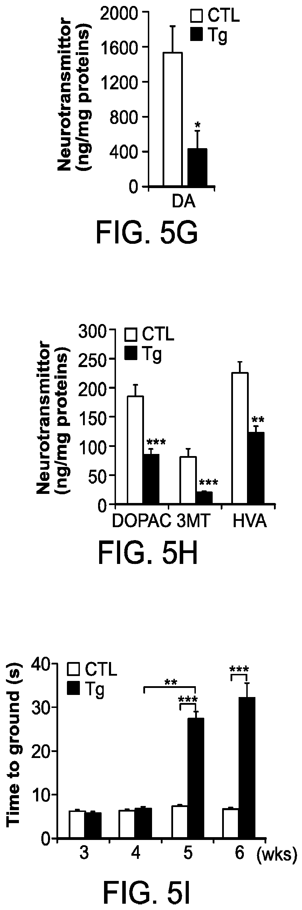

To determine whether farnesol can protect dopaminergic neurons against increased PARIS expression and decreased PGC-1α levels *in vivo,* Tet inducible conditional PARIS transgenic mice (Tg-PARIS) were created. A C-terminal FLAG tagged human PARIS under the control of a tetracycline responsive regulator (TetP-PARIS-Flag) was used to generate tetracycline responsive transgenic mice (Figure S5A). 29 founders expressing TetP-PARIS were identified via PCR screening for the tetracycline promoter (Figure S5B). Three of the highest copy number male founder mice were crossed with the *CamKll*α*-tTA* transgenic mice to achieve transgene expression throughout the mouse forebrain (Mayford et al., 1996). No mice expressing both CamKIIα-tTA and PARIS were born, suggesting that PARIS overexpression during development is embryonic lethal. Thus, dams were maintained on doxycycline to suppress PARIS expression and pups were maintained on doxycycline diet for three weeks after birth. Under these conditions, mice expressing both CamKIIα-tTA and PARIS were identified by PCR (Figure S5C). At 5 weeks of age, overexpression of PARIS is detected in the olfactory bulb (OB), cortex (CTX) , striatum (STR), and ventral midbrain (VM) of Tg-PARIS compared to littermate controls expressing either *CamKIIα-tTA* or *TetP-PARIS* alone (control - CTL) (Figure 5A). In Tg-PARIS line #158, PARIS is overexpressed 15-20 fold in the OB, CTX, and STR (Figure 5A and 5B). In the VM, PARIS is expressed 3 fold, similar to the enhanced expression of PARIS in the conditional parkin knockout mice and in human postmortem brain from sporadic PD patients (Shin et al., 2011) (Figure 5B). The overexpression of PARIS is tetracycline responsive as doxycycline administration completely blocks the upregulation of PARIS (Figure S5D). Two additional independent Tg-PARIS lines (line #121 and line #158) express PARIS at equivalent levels in the CTX and VM (Figure S5E). All three lines die 3 weeks post induction (6 weeks of age) following the development of clasping, seizures and immobility. To confirm that PARIS is expressed in dopamine neurons, co-immunostaining was performed with tyrosine hydroxylase (TH) and FLAG antibodies (Figure 5C). PARIS as indicated by FLAG immunostaining colocalizes with TH positive neurons throughout the SN. Additionally, expression of the CamKII promoter in dopamine neurons was confirmed by crossing the *CamKIIα-tTA* mice with tetP-LacZ reporter mice (Figure S5G). Although the CamKII promoter drives at high levels in the hippocampus (HIP) and forebrain, it drives expression of the LacZ reporter in the VM and SN (Figure 5A~5C and Figure S5G). Dopamine neuronal number was assessed at 6 weeks of age when the mice were moribund by unbiased stereology counting of TH and Nissl positive neurons. There is a greater than 30% reduction of dopamine neurons and a 45% reduction in striatal TH immunoreactivity in Tg-PARIS line #158 (Figure 5D~5F). There is an approximate 25% reduction of dopamine neurons in Tg-PARIS line #121. There is no neuronal loss in the CTX of Tg-PARIS, indicating that the loss of SN dopamine neurons induced by PARIS overexpression is selective for dopamine neurons. Accompanying the loss of dopamine neurons in Tg-PARIS line #158 is a reduction of dopamine and its metabolites in the striatum as determined by HPLC (Figure 5G and 5H). The measurement of dopamine turnover ratio showed that dopamine catabolism in the STR of Tg-PARIS is significantly increased as compared to that of CTL mice. The levels of norepinephrine and epinephrine were unchanged. At 5-6 weeks of age, there is a significant delay in time to descend on the Pole Test, a sensitive measure of dopamine function (Figure 5I).

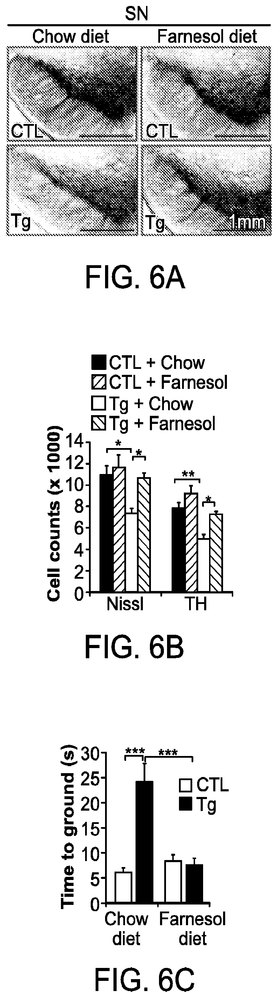

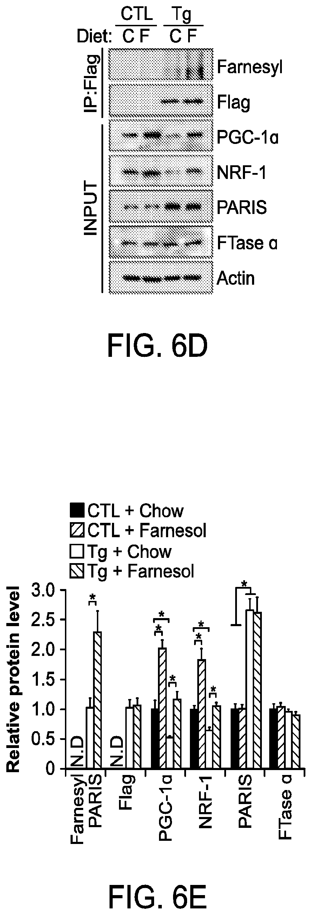

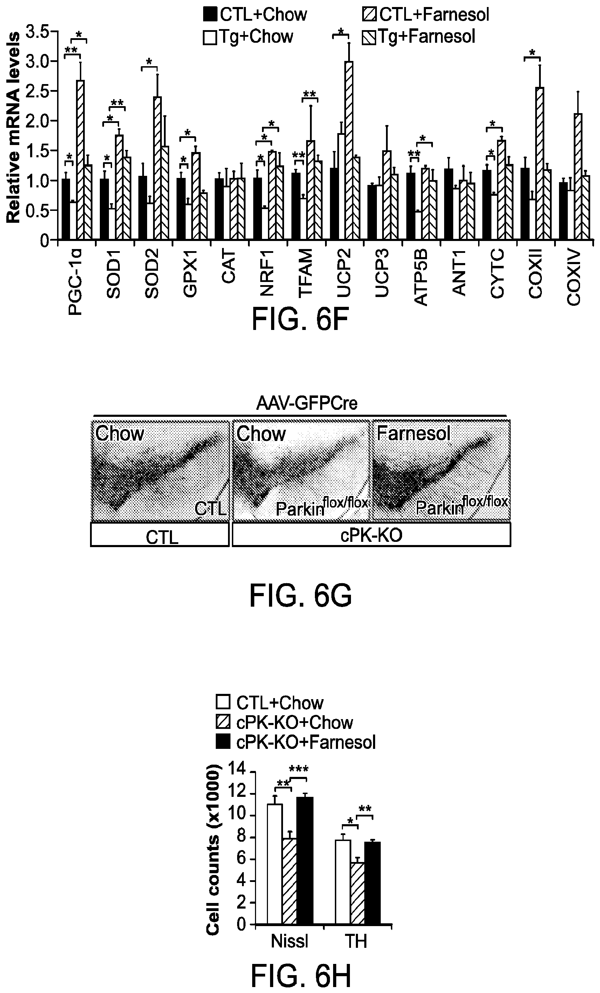

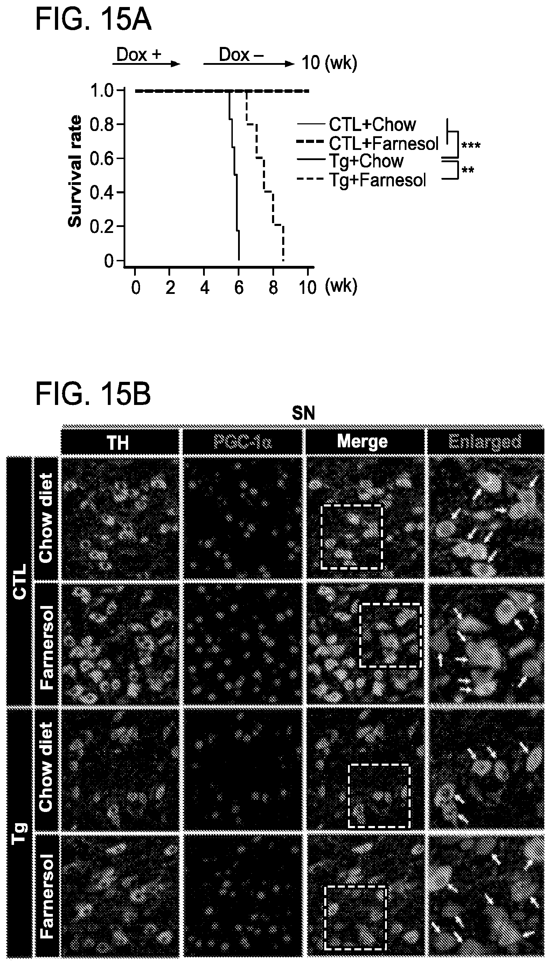

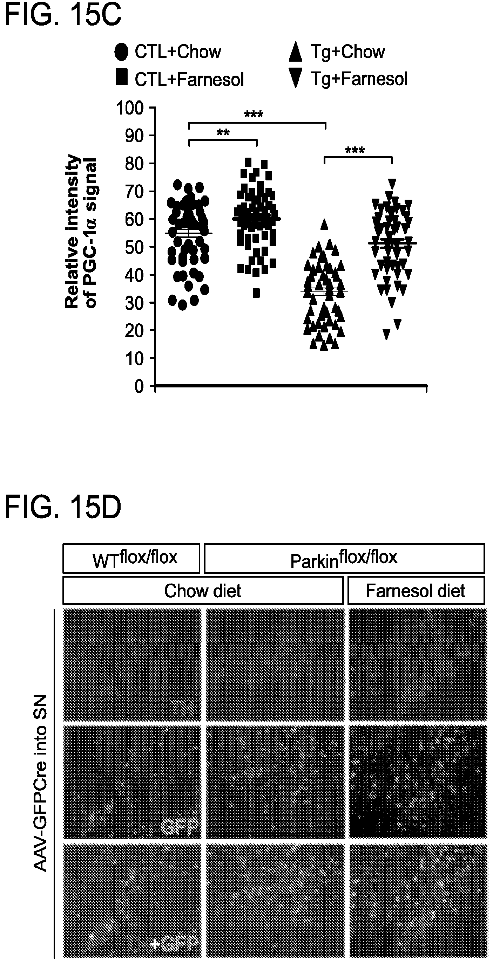

Utilizing this new mouse model, the potential protective effects of farnesol treatment was evaluated. Tg-PARIS mice were either fed a farnesol diet 3 days prior to doxycycline withdrawal. A second cohort of Tg-PARIS mice were maintained on normal mouse chow diet prior to doxycycline withdrawal. Two groups of nontransgenic control (*CamKIIα-tTA* or *TetP-PARIS*) mice were subjected to the same dietary regimen. To determine whether farnesol protects against the loss of dopamine neurons, TH and Nissl stereology was performed. Farnesol treatment completely prevents the loss of dopamine neurons in the SN of Tg-PARIS (Figure 6A and 6B). In addition, there is a complete behavioral rescue as assessed by the Pole Test (Figure 6C). Moreover, farnesol delays the premature lethality due to PARIS overexpression (Figure S6A). Tg-PARIS mice on normal mouse chow exhibit an approximate 50% reduction in PGC-1α levels and a 40% reduction in NRF-1 levels that are blocked by farnesol treatment (Figure 6D and 6E). Farnesol treatment of control mice increases PGC-1α and NRF-1 levels (Figure 6D and 6E). Farnesol treatment leads to enhanced farnesylation of PARIS as detected by immunoprecipitation with FLAG and probing with an antibody to farnesyl (Figure 6D and 6E). In addition, mRNA levels of PGC-1α and a variety of PGC-1α's target genes were measured in the SN of farnesol-fed mice compared to normal chow-fed mice. The mRNA level of PGC-1α and NRF-1 are significantly upregulated in the farnesol-fed mice (Figure 6F). In order to ascertain whether the reduced level of PGC-1α in the dopaminergic neuron of Tg-PARIS SN is restored by farnesol, co-immunostaining analysis was applied, indicating that farnesol led to increased dopaminergic neuronal PGC-1α immunoreactivity (Figure 15B and 15C).

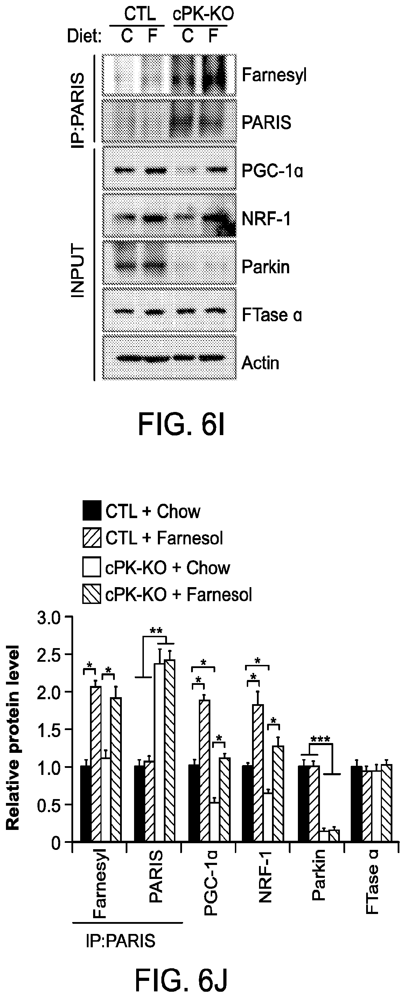

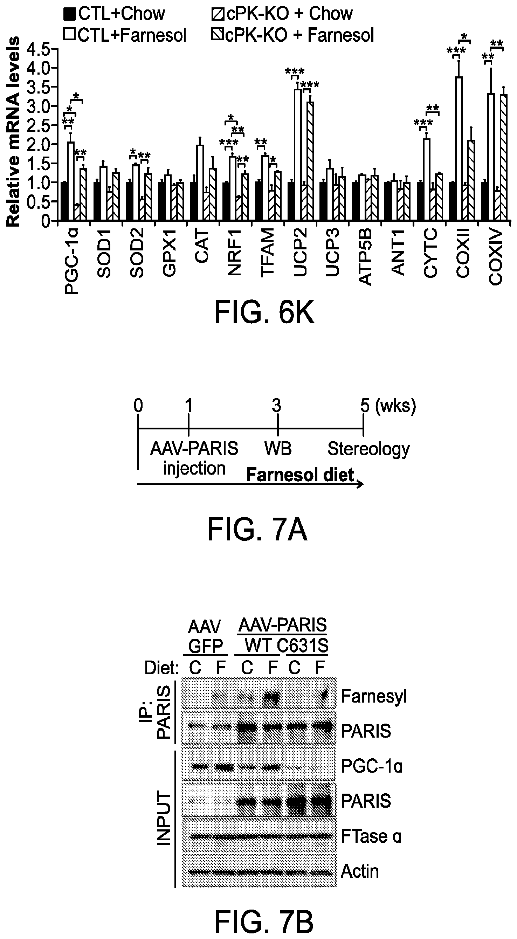

Next, the potential protective effects of farnesol treatment was evaluated in conditional adult parkin knockout (cPK-KO) mice, which have a progressive loss of dopamine neurons that is PARIS-dependent. cPK-KO were generated by stereotaxic injection of AAV-GFPCre into the SN of Parkin^{flox/flox} mice. The cPK-KO mice were fed with either the farnesol diet or the normal mouse chow diet 7 days prior to viral injection until analysis. Wild-type Parkin^{flox/flox} littermates (WT^{flox/flox}) were used as controls and were injected with AAV-GFPCre fed with either the farnesol diet or the normal mouse chow diet 7 days prior to viral injection until analysis. Farnesol treatment completely and significantly prevents the loss of dopamine neurons observed in the SN of cPK-KO mice (Figure 6G and 6H). In this setting, the level of farnesylated PARIS was monitored in the SN of cPK-KO mice fed with either chow diet or farnesol diet. There is a greater than 2 fold increase of PARIS levels in the cPK-KO mice, equivalent to that observed in the SN of sporadic PD (Figure 6I and 6J). cPK-KO mice on normal mouse chow exhibit a reduction of PGC-1α and NRF-1 levels similar to that observed in the Tg-PARIS mice (Figure 6I and 6J). Farnesol treatment increases PGC-1α and NRF-1 levels in the SN of control mice and leads to enhanced farnesylation of PARIS and prevents the decrements of PGC-1α and NRF-1 levels in the SN of cPK-KO mice (Figure 6I and 6J). mRNA levels of PGC-1α and a variety of PGC-1α's target genes were measured in the SN of farnesol-fed mice compared to normal chow-fed mice and find an upregulation of PGC-1α, NRF-1, SOD2, TFAM, UCP2, CytC, COX II and COX 1 demonstrating that farnesol elevates PGC-1α and many of its target genes (Figure 6K).

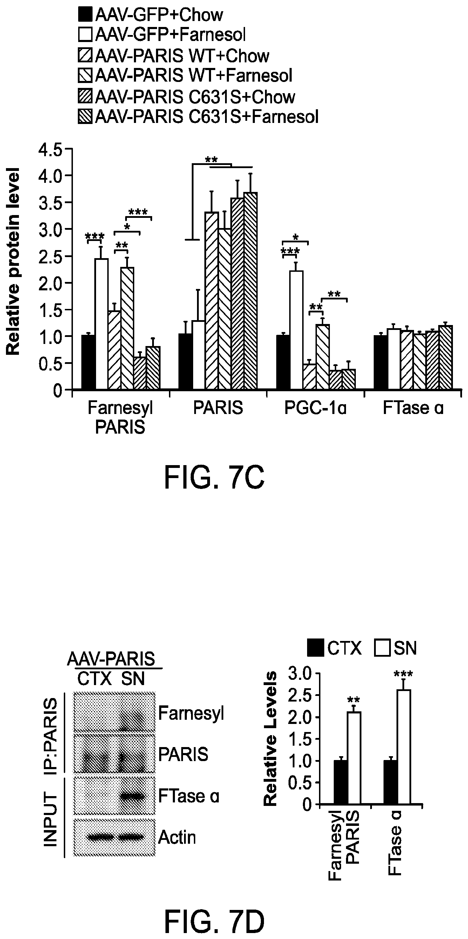

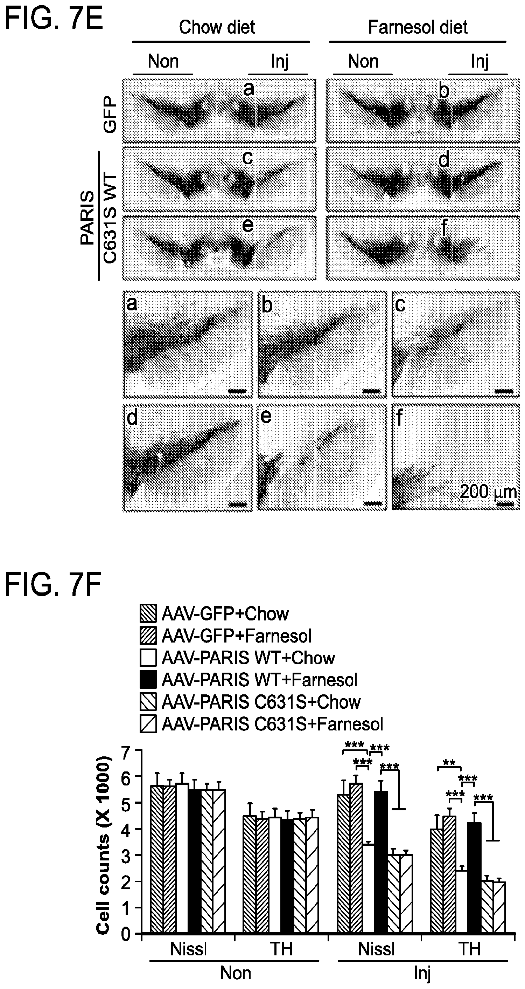

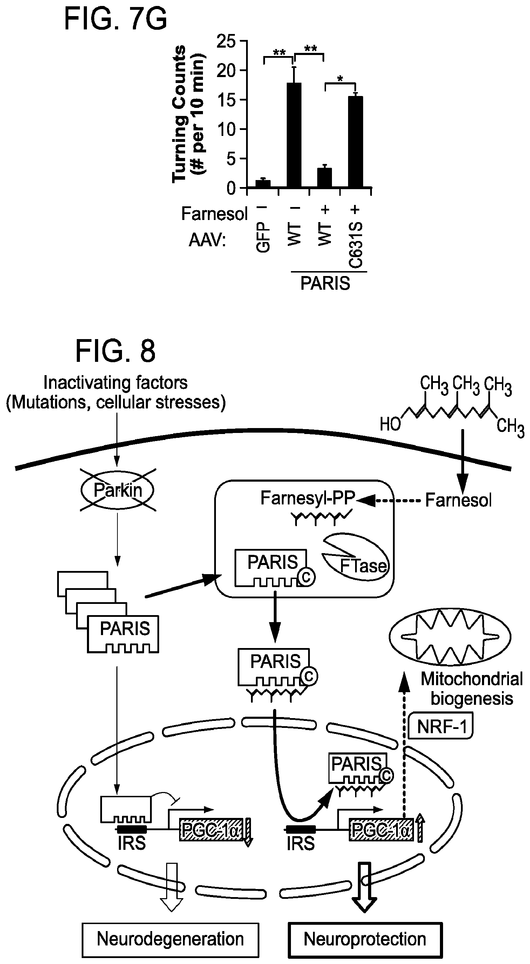

To determine whether the farnesylation of PARIS is required for the protective effects of farnesol *in vivo,* AAV-PARIS WT, AAV-PARIS C631S or AAV-GFP were stereotaxically injected into the SN of C57BL/6J mice. Mice were either fed a normal mouse chow or farnesol diet for one week prior to stereotaxic injection of AAV-PARIS WT, AAV-PARIS C631S or AAV-GFP (Figure 7A). Equivalent expression of transduced AAV vectors was confirmed. Both AAV-PARIS WT and AAV-PARIS C631S lead to a 3-4 fold increase in the expression of PARIS equivalent to that observed in conditional parkin knockout mice and the substantia nigra in sporadic PD (Figure 7B and 7C). Farnesol did not affect the levels of PARIS WT or PARIS C631S (Figure 7B and 7C). Two weeks post-AAV injection in farnesol fed mice farnesylation of PARIS was assessed by probing immunoprecipitated PARIS with an antibody against farnesyl. Farnesylated PARIS is robustly observed in AAV-PARIS WT injected mice as compared to AAV-PARIS C631S injected mice (Figure 7B and 7C). Minimal level of farnesylated PARIS in AAV-PARIS C631S injected mice resulted from endogenous PARIS. AAV-PARIS WT was injected into the CTX and SN of farnesol-fed mice to evaluate if there is region-specific PARIS farnesylation. Interestingly PARIS farnesylation in the SN is highly consistent with the robust level of FTase in the SN, whereas farnesylation in the CTX is low consistent with the low level of FTase in the CTX (Figure 7D). Both AAV-PARIS WT and AAV-PARIS C631S lead to loss of dopamine neurons as assessed by TH and Nissl stereology (Figure 7E and 7F). Farnesol treatment completely prevents the loss of dopamine neurons in the AAV-PARIS WT mice similar to its protection in the Tg- PARIS mice and adult cPK-KO, but it has no effect on dopamine neuronal survival in the AAV-PARIS C631S mice (Figure 7E and 7F). Accompanying the dopaminergic neuronal rescue by farnesol is a restoration of amphetamine-induced rotation behavior in the AAV-PARIS WT (Figure 7G).

The major findings of this invention are that farnesol (3,7,11-trimethyl-2,6,10-dodecatriene-1-ol), a 15-carbon sesquiterpenoid molecule is a potent inducer of PGC-1α through farnesylation and inhibition of PARIS. Screening for PGC-1α inducers followed by a secondary screen of PGC-1α inducers in the setting of PARIS expression led to the identification of several compounds that prevented the repression of PGC-1α by PARIS. Farnesol was selected for further characterization since it had ADME properties suggesting it could be a viable orally bioavailable and brain permeable compound that could modify the levels of PGC-1α. Oral administration of farnesol in mouse chow effectively permeates the BBB to enhance PGC-1α levels. Exploration of the mechanism by which farnesol enhances PGC-1α levels led to the discovery that PARIS is farnesylated on cysteine 631. Farnesylation of PARIS prevents its ability to bind chromatin and inhibit gene transcription. The inhibition of PARIS by farnesol leads to protection against loss of dopamine neurons induced by PARIS over expression in models of PD.

Farnsylation and geranylgeranylation (protein prenylation) is a lipid modification involving the covalent addition of either farnesyl or geranylgeranyl isoprenoids to conserved cysteine residues at the c-terminus of many proteins. A large number of proteins are known to be prenylated, where prenylation is involved in protein targeting to membranes, protein - protein interactions and as a consequence changes the proteins cellular activity.

PARIS interacts with FTase, which accounts for its farnesylation. To our knowledge, the regulation of transcription by protein prenylation is unprecedented. Farnesylation of the Arabidopsis MADS box transcription factor APETALA1 (AP1) has been described, but its role in the function and specificity of AP1 needs to be determined. Farnesylation of PARIS prevents its binding to chromatin where it is unable to influence gene transcription is a unique mechanism of transcriptional control. It will be important to determine where other transcription regulators are controlled by protein prenylation.

Notably it was found that overexpression of PARIS via a conditional transgenic approach at levels equivalent to those observed in the conditional parkin knockout mice and human postmortem substantia nigra from sporadic PD leads to selective loss of dopamine neurons. The neuronal degeneration in PARIS transgenic mice is confined to dopamine neurons in the SN when compared to other brain regions such as cortex, which did not exhibit degeneration despite much higher levels of PARIS. Previously it was shown that AAV-mediated overexpression of PARIS leads to loss of DA neurons that is rescued by parkin or PGC-1α overexpression. Interestingly the PARIS transgenic mice died prematurely. The cause of the early lethality is not known, but is likely due to expression of PARIS in areas directed by CamKIIα promoter. Farnesol completely prevents the loss of DA neurons and partially rescues the early lethality of the PARIS transgenic mice suggesting at least, in part, that the reduction in PGC-1α may account for the early lethality as well as the loss of dopamine neurons. Farnesol protective effects are through inhibition of PARIS through preventing its transcriptional repression by enhancing it farnesylation. Consistent with this notion is the observation that farnesol is not capable of protecting against the farnesylation deficient mutant PARIS 631S. Farnesol also completely prevents the loss of dopamine neurons due to adult conditional knockout of parkin. Since deletion of PARIS prevents the loss of dopamine neurons in adult conditional parkin knockout mice through preventing decrements in PGC-1α and the deleterious consequences of decreased PGC-1α, farnesol's enhancement of PARIS farnesylation and restoration of PGC-1α levels and PGC-1α target genes in adult conditional parkin knockout mice likely accounts for the protective actions of farnesol.

Farnesol is a known agonist for the Farensoid X receptor (FXR, also known as NR1H4). Since others and we have shown that FXR is not expressed in the brain, the effect of farnesol reported here is likely FXR-independent. Farnesol is also capable of activating both PPARα and PPARγ *in vitro, where it has more potent effects on* PPARα. No effects of farnesol on PPARα and PPARγ in mouse brain and SH-SY5Y cells were observed, indicating the actions of farnesol on PGC-1α in the brain is PPARα and PPARγ - independent. Moreover, since farnesol enhances the levels of PGC-1α through farnesylation of PARIS and fails to increase PGC-1α levels in the absence of PARIS and since PPARγ is not known to regulate the levels of PGC-1α, farnesol actions on PGC-1α are most likely through its farneslyation of PARIS and independent from its actions on PPARs.

Dietary farnesol in rats at similar concentrations as used here has been studied in the context of carbohydrate and lipid metabolism where it lowers the level of serum triglycerides, cholesterol via its action on FXR and PPARα. Dietary farnesol in rodents also exhibits cardioprotective effects independent of its actions on FXR and PPARs through enhancing protein geranylgeranylation. Farnesol is normally found in herbs, berries and fruits which might account, in part, for their beneficial effects on disorders of lipid metabolism and cardiovascular health. Farnesol can be phosphorylated *in vivo* to form FPP where it can enhance protein farnesylation and geranylgeranylation. Thus, farnesol enhancement of protein prenylation seems to be a major mechanism of it beneficial effects in both the brain and heart. Farnesol is also a common additive in cigarettes. Perhaps farnesol could be the constituent in cigarette smoke that accounts for the epidemiologic data that cigarette smoking is protective against the development of PD. It will be important to determine the amount of farnesol that can be safely tolerated in humans as well as its pharmacokinetic properties and whether farnesol protects against the degenerative effects of PD in humans.

### METHODS/EXAMPLES

### Generation of Reporter Cell line and Chemical Screening.

For stable reporter SH-SY5Y cells (SH-PGC-1α-Luc), 1-Kb human PGC-1α promoter was inserted into lentiviral pGreenFire (System Biosciences). SH-SY5Y cells were transduced with concentrated virus and selected with puromycin (1 µg/ml) as described in the supplemental information. SH-PGC-1α-Luc cells were plated into 96-well white, flat-bottom plates at 10,000 cell/well in 100 µl of DMEM and incubated overnight at 37 ° C, 5% CO₂. The next day, compounds were treated and luciferase activity was measured using SteadyGlo reagent (Promega) after 48 hrs incubation. Each plate had three internal controls, daidzein (as a positive, control 10 µM) and two negative controls (no treatment and DMSO). The raw luciferase data for each well were normalized as described in the supplemental information.

### Plasmid Constructions.

pCMV-PARIS wild-type (pCMV-Tag2A, Stratagene) was generated as described (Shin et al., 2011) and pCMV-PARIS C631S mutant was generated using a QuikChange site-directed mutagenesis kit (Stratagene). More details are available in the supplemental information.

### In vitro farnesylation assay.

The *in vitro* farnesylation assay (IVF) was performed as described (Nguyen et al., 2009; Nguyen et al., 2010; Wu et al., 2007). Briefly, for metabolic global farnesylation, 5 µCi [³H]-farnesyl pyrophosphate (FPP) (NET1042001MC, PerkinElmer) was treated into SH-SY5Y cells and immunoprecipitant was monitored by immunoblot analysis with Farnesyl antibody. For artificial IVF, the cell lysate was incubated for 6 h with biotin-geranyl pyrophosphate (Biotin-GPP, specific substrate of RabGGTase, LI-015, Jena Bioscience) and recombinant FTase W102T/Y154T mutant (PR-952, Jena Bioscience). For fluorescence-based IVF, immunoprecipitated Flag-tagged PARIS was incubated for 20 min with NBD-GPP (LI-014, Jena Bioscience). After SDS-PAGE, the gel was scanned by a fluorescent imager (FluorChem ^{™} Q system). More details are available in the supplemental information.

### CAST, EMSA, ChIP, qRT-PCR Assays

GST, GST-PARIS, GST-PARIS-C631S were used for electrophoretic mobility shift assays (EMSA) as described in the supplemental information. Chromatin immunoprecipitation was carried out according to the manufacturer's instruction as described in the supplemental information.

### Conditional PARIS transgenic and Conditional Parkin Knockout mice

To generate tet-off conditional PARIS transgenic mice, linearized transgenic constructs (Notl digestion, 8 kbp) were microinjected into the embryos and the embryos were transferred into B6D2F1 pseudo-pregnant female mice (Transgenic Animal Core of National Cancer Institute). The three high copy founders (line # 121, 158, 179) were mated with C57/BL6 mice for two to three generations to establish the transgenic lines. PARIS induction in conditional transgenic mice was suppressed by feeding the mice with doxycycline-containing food (doxycycline Diet-Sterile, 200 mg per kg doxycycline, Bio-Serv).

To generate Cre-flox conditional model of parkin knock out, an adenoassociated vector expressing GFP fused Cre recombinase (AAV-GFPCre) was stereotaxically introduced into the SN of exon 7 floxed parkin mice (parkin^{Flox/Flox}).

### Statistics

In general, quantitative data are presented as the mean ± SEM. Statistical significance was either assessed via an unpaired two-tailed Student's t test or an ANOVA test with Student-Newman-Keuls post hoc analysis. Differences were considered significant when *p* < 0.05.

### Pharmaceutical Preparations

Pharmaceutical compositions of the present invention comprise an effective amount of one or more inducers of expression of peroxisome proliferator-activated receptor-γ coactivator-1α (PGC-1α)selected from the group consisting of farnesol, ABT-0529 and a combination thereof, or a pharmaceutically acceptable salt, solvate, phosphate derivative, or stereoisomer thereof, dissolved or dispersed in a pharmaceutically acceptable carrier. The phrases "pharmaceutical or pharmacologically acceptable" refers to molecular entities and compositions that do not produce an adverse, allergic or other untoward reaction when administered to an animal, such as, for example, a human, as appropriate. The preparation of a pharmaceutical composition that comprises at least one inducer of expression of PGC-1α or additional active ingredient will be known to those of skill in the art in light of the present disclosure, as exemplified by Remington: The Science and Practice of Pharmacy, 21st Ed. Lippincott Williams and Wilkins, 2005. Moreover, for animal (e.g., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the pharmaceutical compositions is contemplated.

The composition may comprise different types of carriers depending on whether it is to be administered in solid, liquid or aerosol form, and whether it need to be sterile for such routes of administration as injection. The present compositions can be administered intravenously, intradermally, transdermally, intrathecally, intraarterially, intraperitoneally, intranasally, intravaginally, intrarectally, topically, intramuscularly, subcutaneously, mucosally, orally, topically, locally, inhalation (e.g., aerosol inhalation), injection, infusion, continuous infusion, localized perfusion bathing target cells directly, *via* a catheter, *via* a lavage, in cremes, in lipid compositions (e.g., liposomes), or by other method or any combination of the forgoing as would be known to one of ordinary skill in the art (see, for example, Remington's Pharmaceutical Sciences, 18th Ed. Mack Printing Company, 1990).

The inducer of expression of PGC-1α may be formulated into the composition in a free base, neutral or salt form. Pharmaceutically acceptable salts, include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Upon formulation, solutions will be administered in a manner compatible with the dosage formulation and in such amount as is therapeutically effective. The formulations are easily administered in a variety of dosage forms such as formulated for parenteral administrations such as injectable solutions, or aerosols for delivery to the lungs, or formulated for alimentary administrations such as drug release capsules and the like.

Further in accordance with the present disclosure, the composition of the present invention suitable for administration is provided in a pharmaceutically acceptable carrier with or without an inert diluent. The carrier should be assimilable and includes liquid, semi-solid, i.e., pastes, or solid carriers. Except insofar as any conventional media, agent, diluent or carrier is detrimental to the recipient or to the therapeutic effectiveness of a composition contained therein, its use in administrable composition for use in practicing the methods of the present invention is appropriate. Examples of carriers or diluents include fats, oils, water, saline solutions, lipids, liposomes, resins, binders, fillers and the like, or combinations thereof. The composition may also comprise various antioxidants to retard oxidation of one or more component. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof. In accordance with the present invention, the composition is combined with the carrier in any convenient and practical manner, *i.e.,* by solution, suspension, emulsification, admixture, encapsulation, absorption and the like. Such procedures are routine for those skilled in the art.

In a specific embodiment of the present invention, the composition is combined or mixed thoroughly with a semi-solid or solid carrier. The mixing can be carried out in any convenient manner such as grinding. Stabilizing agents can be also added in the mixing process in order to protect the composition from loss of therapeutic activity, i.e., denaturation in the stomach. Examples of stabilizers for use in the composition include buffers, amino acids such as glycine and lysine, carbohydrates such as dextrose, mannose, galactose, fructose, lactose, sucrose, maltose, sorbitol, mannitol, *etc.*

In further embodiments, the present invention may concern the use of a pharmaceutical lipid vehicle composition that includes the inducer of expression of PGC-1α, one or more lipids, and an aqueous solvent. As used herein, the term "lipid" will be defined to include any of a broad range of substances that is characteristically insoluble in water and extractable with an organic solvent. This broad class of compounds are well known to those of skill in the art, and as the term "lipid" is used herein, it is not limited to any particular structure. Examples include compounds which contain long-chain aliphatic hydrocarbons and their derivatives. A lipid may be naturally occurring or synthetic (*i.e*., designed or produced by man). However, a lipid is usually a biological substance. Biological lipids are well known in the art, and include for example, neutral fats, phospholipids, phosphoglycerides, steroids, terpenes, lysolipids, glycosphingolipids, glycolipids, sulphatides, lipids with ether and ester-linked fatty acids and polymerizable lipids, and combinations thereof. Of course, compounds other than those specifically described herein that are understood by one of skill in the art as lipids are also encompassed by the compositions and methods of the present invention.

One of ordinary skill in the art would be familiar with the range of techniques that can be employed for dispersing a composition in a lipid vehicle. For example, the inducer of expression of PGC-1α may be dispersed in a solution containing a lipid, dissolved with a lipid, emulsified with a lipid, mixed with a lipid, combined with a lipid, covalently bonded to a lipid, contained as a suspension in a lipid, contained or complexed with a micelle or liposome, or otherwise associated with a lipid or lipid structure by any means known to those of ordinary skill in the art. The dispersion may or may not result in the formation of liposomes.

The actual dosage amount of a composition of the present invention administered to a subject can be determined by physical and physiological factors such as body weight, severity of condition, the type of disease being treated, previous or concurrent therapeutic interventions, idiopathy of the patient and on the route of administration. Depending upon the dosage and the route of administration, the number of administrations of a preferred dosage and/or an effective amount may vary according to the response of the subject. The practitioner responsible for administration will, in any event, determine the concentration of active ingredient(s) in a composition and appropriate dose(s) for the individual subject.

In certain embodiments, pharmaceutical compositions may comprise, for example, at least about 0.1% of an active compound. In other embodiments, the active compound may comprise between about 2% to about 75% of the weight of the unit, or between about 25% to about 60%, for example, and any range derivable therein. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 500 milligram/kg/body weight, etc., can be administered, based on the numbers described above.

### A. Alimentary Compositions and Formulations

In one embodiment of the present disclosure, compositions comprising the inducers of expression of PGC-1α are formulated to be administered *via* an alimentary route. Alimentary routes include all possible routes of administration in which the composition is in direct contact with the alimentary tract. Specifically, the pharmaceutical compositions disclosed herein may be administered orally, buccally, rectally, or sublingually. As such, these compositions may be formulated with an inert diluent or with an assimilable edible carrier, or they may be enclosed in hard- or soft- shell gelatin capsule, or they may be compressed into tablets, or they may be incorporated directly with the food of the diet.

In certain embodiments, the active compounds may be incorporated with excipients and used in the form of ingestible tablets, buccal tables, troches, capsules, elixirs, suspensions, syrups, wafers, and the like (Mathiowitz et al., 1997; Hwang et al., 1998; U.S. Pat. Nos. 5,641,515; 5,580,579 and 5,792, 451). The tablets, troches, pills, capsules and the like may also contain the following: a binder, such as, for example, gum tragacanth, acacia, cornstarch, gelatin or combinations thereof; an excipient, such as, for example, dicalcium phosphate, mannitol, lactose, starch, magnesium stearate, sodium saccharine, cellulose, magnesium carbonate or combinations thereof; a disintegrating agent, such as, for example, corn starch, potato starch, alginic acid or combinations thereof; a lubricant, such as, for example, magnesium stearate; a sweetening agent, such as, for example, sucrose, lactose, saccharin or combinations thereof; a flavoring agent, such as, for example peppermint, oil of wintergreen, cherry flavoring, orange flavoring, etc. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. When the dosage form is a capsule, it may contain, in addition to materials of the above type, carriers such as a liquid carrier. Gelatin capsules, tablets, or pills may be enterically coated. Enteric coatings prevent denaturation of the composition in the stomach or upper bowel where the pH is acidic. See, e.g., U.S. Pat. No. 5,629,001. Upon reaching the small intestines, the basic pH therein dissolves the coating and permits the composition to be released and absorbed by specialized cells, e.g., epithelial enterocytes and Peyer's patch M cells. A syrup of elixir may contain the active compound sucrose as a sweetening agent methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations.

For oral administration the compositions of the present disclosure may alternatively be incorporated with one or more excipients in the form of a mouthwash, dentifrice, buccal tablet, oral spray, or sublingual orally- administered formulation. For example, a mouthwash may be prepared incorporating the active ingredient in the required amount in an appropriate solvent, such as a sodium borate solution (Dobell's Solution). Alternatively, the active ingredient may be incorporated into an oral solution such as one containing sodium borate, glycerin and potassium bicarbonate, or dispersed in a dentifrice, or added in a therapeutically- effective amount to a composition that may include water, binders, abrasives, flavoring agents, foaming agents, and humectants. Alternatively, the compositions may be fashioned into a tablet or solution form that may be placed under the tongue or otherwise dissolved in the mouth.

Additional formulations which are suitable for other modes of alimentary administration include suppositories. Suppositories are solid dosage forms of various weights and shapes, usually medicated, for insertion into the rectum. After insertion, suppositories soften, melt or dissolve in the cavity fluids. In general, for suppositories, traditional carriers may include, for example, polyalkylene glycols, triglycerides or combinations thereof. In certain embodiments, suppositories may be formed from mixtures containing, for example, the active ingredient in the range of about 0.5% to about 10%, and preferably about 1% to about 2%.

### B. Parenteral Compositions and Formulations

In further embodiments, compositions comprising the inducer of expression of PGC-1α may be administered via a parenteral route. As used herein, the term "parenteral" includes routes that bypass the alimentary tract. Specifically, the pharmaceutical compositions disclosed herein may be administered for example, but not limited to intravenously, intradermally, intramuscularly, intraarterially, intrathecally, subcutaneous, or intraperitoneally U.S. Pat. Nos. 6,7537,514, 6,613,308, 5,466,468, 5,543,158; 5,641,515; and 5,399,363.

Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms. The pharmaceutical forms suitable for injectable use include sterile aqueous solutions or dispersions and sterile powders for the extemporaneous preparation of sterile injectable solutions or dispersions (U.S. Patent 5,466,468). In all cases the form must be sterile and must be fluid to the extent that easy injectability exists. It must be stable under the conditions of manufacture and storage and must be preserved against the contaminating action of microorganisms, such as bacteria and fungi. The carrier can be a solvent or dispersion medium containing, for example, water, ethanol, polyol (*i.e.*, glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous, and intraperitoneal administration. In this connection, sterile aqueous media that can be employed will be known to those of skill in the art in light of the present disclosure. For example, one dosage may be dissolved in isotonic NaCl solution and either added hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.
Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. A powdered composition is combined with a liquid carrier such as, e.g., water or a saline solution, with or without a stabilizing agent.

### C. Miscellaneous Pharmaceutical Compositions and Formulations

In other preferred embodiments of the invention, compositions comprising the active compound inducer of expression of PGC-1α may be formulated for administration via various miscellaneous routes, for example, topical (*i.e.*, transdermal) administration, mucosal administration (intranasal, vaginal, *etc*.) and/or inhalation.

Pharmaceutical compositions for topical administration may include the active compound formulated for a medicated application such as an ointment, paste, cream or powder. Ointments include all oleaginous, adsorption, emulsion and water-solubly based compositions for topical application, while creams and lotions are those compositions that include an emulsion base only. Topically administered medications may contain a penetration enhancer to facilitate adsorption of the active ingredients through the skin. Suitable penetration enhancers include glycerin, alcohols, alkyl methyl sulfoxides, pyrrolidones and luarocapram. Possible bases for compositions for topical application include polyethylene glycol, lanolin, cold cream and petrolatum as well as any other suitable absorption, emulsion or water-soluble ointment base. Topical preparations may also include emulsifiers, gelling agents, and antimicrobial preservatives as necessary to preserve the active ingredient and provide for a homogenous mixture. Transdermal administration of the present invention may also comprise the use of a "patch". For example, the patch may supply one or more active substances at a predetermined rate and in a continuous manner over a fixed period of time.

In certain embodiments, the pharmaceutical compositions may be delivered by eye drops, intranasal sprays, inhalation, and/or other aerosol delivery vehicles. Methods for delivering compositions directly to the lungs via nasal aerosol sprays has been described e.g., in U.S. Pat. Nos. 5,756,353 and 5,804,212. Likewise, the delivery of drugs using intranasal microparticle resins (Takenaga et al., 1998) and lysophosphatidyl-glycerol compounds (U.S. Pat. No. 5,725, 871) are also well-known in the pharmaceutical arts. Likewise, transmucosal drug delivery in the form of a polytetrafluoroetheylene support matrix is described in U.S. Pat. No. 5,780,045.

The term aerosol refers to a colloidal system of finely divided solid of liquid particles dispersed in a liquefied or pressurized gas propellant. The typical aerosol of the present invention for inhalation will consist of a suspension of active ingredients in liquid propellant or a mixture of liquid propellant and a suitable solvent. Suitable propellants include hydrocarbons and hydrocarbon ethers. Suitable containers will vary according to the pressure requirements of the propellant. Administration of the aerosol will vary according to subject's age, weight and the severity and response of the symptoms.

### Kits of the Disclosure

Any of the compositions described herein may be comprised in a kit.

The kits may comprise a suitably aliquoted composition of the invention and, in some cases, one or more additional agents. The component(s) of the kits may be packaged either in aqueous media or in lyophilized form. The container means of the kits will generally include at least one vial, test tube, flask, bottle, syringe or other container means, into which a component may be placed, and preferably, suitably aliquoted. Where there is more than one component in the kit, the kit may also contain a second, third or other additional container into which the additional components may be separately placed. However, various combinations of components may be comprised in a vial. The kits may also include a means for containing the composition of the invention and any other reagent containers in close confinement for commercial sale. Such containers may include injection or blow-molded plastic containers into which the desired vials are retained.

When the components of the kit are provided in one and/or more liquid solutions, the liquid solution is an aqueous solution, with a sterile aqueous solution being particularly preferred. The inducer of expression of PGC-1α composition(s) may be formulated into a syringeable composition. In which case, the container means may itself be a syringe, pipette, and/or other such like apparatus, from which the formulation may be applied to an infected area of the body, injected into an animal, and/or even applied to and/or mixed with the other components of the kit. However, the components of the kit may be provided as dried powder(s). When reagents and/or components are provided as a dry powder, the powder can be reconstituted by the addition of a suitable solvent. It is envisioned that the solvent may also be provided in another container means.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A composition comprising one or more inducers of expression of peroxisome proliferator-activated receptor-y coactivator-1α (PGC-1α) for use in preventing or treating Parkinson's disease in a subject, wherein the inducer is an entity selected from the group consisting of Farnesol, ABT-0529 and a combination thereof, or a pharmaceutically acceptable salt, solvate, phosphate derivative, or stereoisomer thereof.

2. The composition for use of claim 1, wherein the inducer is Farnesol or a pharmaceutically acceptable salt, solvate, phosphate derivative, or stereoisomer thereof.

3. The composition for use of claim 2, wherein the composition is for administration in an amount that: (i) results in an increase of the mRNA level of NRF-1 in the subject; (ii) increases farnesyl transferase activity in the brain of the subject; or (iii) increases parkin interacting substrate (PARIS) farnesylation in the brain of the subject, compared to when the subject is not administered the composition.

4. The composition for use of claim 3, wherein the farnesylation occurs on a cysteine residue at residue 631 of PARIS.

5. The composition for use of any one of claims 1 to 4, wherein the composition substantially eliminates the PARIS binding to its target DNA in the subject when compared to the subject that is not administered the composition.

6. The composition for use of any one of claims 1 to 5, wherein the composition prevents the loss of dopamine neurons in the subject when compared to the subject that is not administered the composition.

7. The composition for use of any one of claims 1 or 4 to 6, wherein the entity is ABT-0529.

## Patentansprüche

1. Zusammensetzung, die einen oder mehrere Induktoren einer Expression des Peroxisomen-Proliferator-aktivierten Rezeptor-γ-Koaktivators 1α (PGC-1α) zur Verwendung bei der Vorbeugung oder Behandlung der Parkinson-Krankheit bei einem Subjekt umfasst, wobei der Induktor eine Einheit ist, die aus der Gruppe ausgewählt ist, die aus Farnesol, ABT-0529 und einer Kombination davon oder einem pharmazeutisch akzeptablen Salz, Solvat, Phosphatderivat oder Stereoisomer davon besteht.

2. Zusammensetzung zur Verwendung nach Anspruch 1, wobei der Induktor Farnesol oder ein pharmazeutisch akzeptables Salz, Solvat, Phosphatderivat oder Stereoisomer davon ist.

3. Zusammensetzung zur Verwendung nach Anspruch 2, wobei die Zusammensetzung zur Verabreichung in einer Menge vorgesehen ist, die: (i) zu einer Erhöhung des mRNA-Spiegels von NRF-1 in dem Subjekt führt; (ii) Farnesyltransferase-Aktivität im Gehirn des Subjekts erhöht; oder (iii) Farnesylierung des mit Parkin interagierenden Substrats (PARIS) im Gehirn des Subjekts im Vergleich zu dem Subjekt, dem die Zusammensetzung nicht verabreicht wird, erhöht.

4. Zusammensetzung zur Verwendung nach Anspruch 3, wobei die Farnesylierung an einem Cysteinrest am Rest 631 von PARIS auftritt.

5. Zusammensetzung zur Verwendung nach einem der Ansprüche 1 bis 4, wobei die Zusammensetzung die PARIS-Bindung an ihre Ziel-DNA in dem Subjekt im Vergleich zu dem Subjekt, dem die Zusammensetzung nicht verabreicht wird, weitgehend eliminiert.

6. Zusammensetzung zur Verwendung nach einem der Ansprüche1 bis 5, wobei die Zusammensetzung den Verlust von Dopaminneuronen bei dem Subjekt im Vergleich zu dem Subjekt, dem die Zusammensetzung nicht verabreicht wird, verhindert.

7. Zusammensetzung zur Verwendung nach einem der Ansprüche 1 oder 4 bis 6, wobei die Einheit ABT- 0529 ist.

## Revendications

1. Composition comprenant un ou plusieurs inducteurs d'expression du coactivateur y 1α de récepteur activé par proliférateur de peroxysome-(PGC-1α) destinée à être utilisée dans la prévention ou le traitement de la maladie de Parkinson chez un sujet, dans laquelle l'inducteur est une entité sélectionnée à partir du groupe constitué de farnésol, ABT-0529 et une combinaison de ceux-ci, ou un sel, solvate, dérivé de phosphate ou stéréoisomère pharmaceutiquement acceptable de ceux-ci.

2. Composition destinée à être utilisée selon la revendication 1, dans laquelle l'inducteur est du farnésol ou un sel, solvate, dérivé de phosphate ou stéréoisomère pharmaceutiquement acceptable de celui-ci.

3. Composition destinée à être utilisée selon la revendication 2, dans laquelle la composition est destinée à être administrée en une quantité qui : (i) entraîne une augmentation du niveau d'ARNm de NRF-1 chez le sujet ; (ii) augmente l'activité de farnésyl transférase dans le cerveau du sujet ; ou (iii) augmente la farnésylation du substrat interagissant avec la parkine (PARIS) dans le cerveau du sujet, par comparaison au sujet auquel la composition n'a pas été administrée.

4. Composition destinée à être utilisée selon la revendication 3, dans laquelle la farnésylation se produit sur un résidu de cystéine au niveau du résidu 631 de PARIS.

5. Composition destinée à être utilisée selon l'une quelconque des revendications 1 à 4, dans laquelle la composition élimine sensiblement la liaison de PARIS à son ADN cible chez le sujet par comparaison au sujet auquel la composition n'a pas été administrée.

6. Composition destinée à être utilisée selon l'une quelconque des revendications 1 à 5, dans laquelle la composition empêche la perte de neurones dopaminergiques chez le sujet par comparaison au sujet auquel la composition n'a pas été administrée.

7. Composition destinée à être utilisée selon l'une quelconque des revendications 1 ou 4 à 6, dans laquelle l'entité est ABT-0529.
